(19) Europäisches Patentamt
European Patent Office
Office européen des brevets

(11) Publication number: **0 058 424**
**B1**

(12) **EUROPEAN PATENT SPECIFICATION**

(45) Date of publication of patent specification: **18.09.85**

(21) Application number: **82101106.1**

(22) Date of filing: **15.02.82**

(51) Int. Cl.⁴: **C 07 D 231/06,** A 01 N 43/56, A 01 N 47/38 // C07C49/84, C07C79/35, C07C93/14, C07C97/10, C07C119/048, C07C121/75, C07C149/34, C07C121/76, C07C149/42

# (54) Pyrazoline derivatives.

(30) Priority: **17.02.81 JP 21767/81**
**19.06.81 JP 94850/81**
**14.01.82 JP 4326/82**

(43) Date of publication of application:
**25.08.82 Bulletin 82/34**

(45) Publication of the grant of the patent:
**18.09.85 Bulletin 85/38**

(84) Designated Contracting States:
**BE CH DE FR IT LI NL**

(56) References cited:
**EP-A-0 004 733**
**EP-A-0 021 506**
**DE-A-2 700 288**
**GB-A-1 398 196**
**GB-A-1 514 285**
**GB-A-1 568 003**
**GB-A-1 570 635**
**NL-A-7 301 203**

**The file contains technical information submitted after the application was filed and not included in this specification**

(73) Proprietor: **NISSAN CHEMICAL INDUSTRIES LTD.**
**7-1, 3-chome Kanda-Nishiki-cho**
**Chiyoda-ku Tokyo (JP)**

(72) Inventor: **Ozawa, Kiyomi**
**Nissan Chemical Ind. Ltd. Chuo Kenkyusho**
**722-1, Tsuboicho Funabashi-shi Chiba-ken (JP)**
Inventor: **Nakajima, Yasuyuki**
**Nissan Chemical Ind. Ltd. Chuo Kenkyusho**
**722-1, Tsuboicho Funabashi-shi Chiba-ken (JP)**
Inventor: **Tsugeno, Makoto**
**Nissan Chemical Ind. Ltd. Chuo Kenkyusho**
**722-1, Tsuboicho Funabashi-shi Chiba-ken (JP)**
Inventor: **Ishii, Shigeru**
**Nissan Chemical Ind. Ltd. Chuo Kenkyusho**
**722-1, Tsuboicho Funabashi-shi Chiba-ken (JP)**
Inventor: **Hatanaka, Masataka**
**Nissan Chemical Ind., Ltd. Chuo Kenkyusho**
**722-1, Tsuboicho Cunabashi-shi Chiba-ken (JP)**
Inventor: **Hirose, Masayoshi Nissan Chemical Ind., Ltd.**
**No. 1470, Oaza-Shiraoka Shiraoka-machi**
**Minami-Saitama-gun Saitama-ken (JP)**
Inventor: **Kudo, Masaki Nissan Chemical Ind., Ltd.**
**No. 1470, Oaza-Shiraoka Shiraoka-machi**
**Minami-Saitama-gun Saitama-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 058 424 B1

(74) Representative: **Wächtershäuser, Günter, Dr.**
**Tal 29**
**D-8000 München 2 (DE)**

**Description**

Field of the Invention

The present invention relates to novel pyrazoline derivatives a production thereof and an insecticide containing a pyrazoline derivative thereof as an active ingredient.

Description of the Prior Art

Various chemicals for insecticides have been studied and developed for a long time. These insecticides have been contributed for improvement of a productivity of agricultural crops. However, a development of a novel chemical having superior insecticidal activity has been required.

It has been known that 1-carbamoyl-2-pyrazoline derivatives are effective as insecticides in Japanese Unexamined Patent Publication No. 87028/1973, No. 41358/1976 and No. 87166/1977, etc. as pyrazoline derivatives. Further, such pyrazoline derivatives are known from GB—A—1568003, EP—A—4733, DE—A—2700288, NL—A 7301203, GB—A—1514285, GB—A—1570635, GB—A—1398196 and EP—A—21506.

Summary of the Invention

It is an object of the present invention to provide novel insecticidal compounds which have excellent effects and low toxicity to mammals and fishes.

It is another object of the present invention to provide a process for producing novel pyrazoline derivatives.

The foregoing and other objects of the present invention have been attained by providing pyrazoline derivatives having the formula

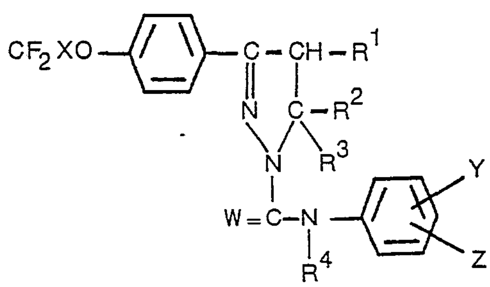

[I]

wherein $R^1$ represents hydrogen atom, a $C_{1-4}$ alkyl group, phenyl group, a halogen-substituted phenyl group, a cyano alkyl group or a $C_{1-4}$ alkoxy alkyl group; $R^2$ and $R^3$ respectively represent hydrogen atom, a $C_{1-4}$ alkyl group, phenyl group or a halogen-substituted phenyl group; $R^4$ represents hydrogen atoms or a $C_{1-4}$ alkyl group; X represents hydrogen atom or a halogen atom; W represents oxygen or sulfur atom; Y and Z respectively represent hydrogen atom, a halogen atom, a $C_{1-4}$ alkyl group, a $C_{1-4}$ alkoxy group, nitro group, trifluoromethyl group, a $C_{1-4}$ alkylthio group, an acetyl group, nitrile group, a $C_{1-4}$ alkyl sufonyl group, a $C_{1-4}$ alkoxycarbonyl group, —A—$R^5$ or Y and Z form

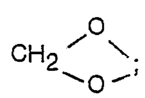

and A represents —O—, —S—, —SO— or —$SO_2$— and $R^5$ represents a halogen-substituted $C_{1-4}$ alkyl group.

Preferred compounds have the following formula (II)

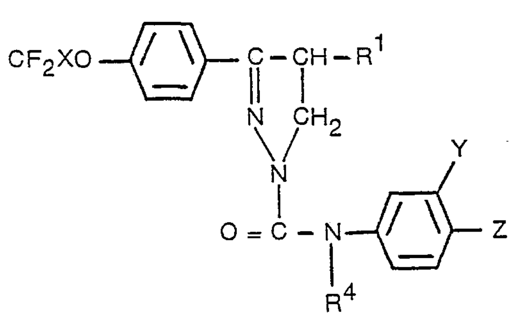

(II)

wherein $R^1$, $R^2$, $R^3$, $R^4$, X, Y and Z have the meanings given above.

Detailed description of the preferred embodiments

The compounds of the present invention have been produced by the following reaction scheme 1 or 2.

Scheme 1:

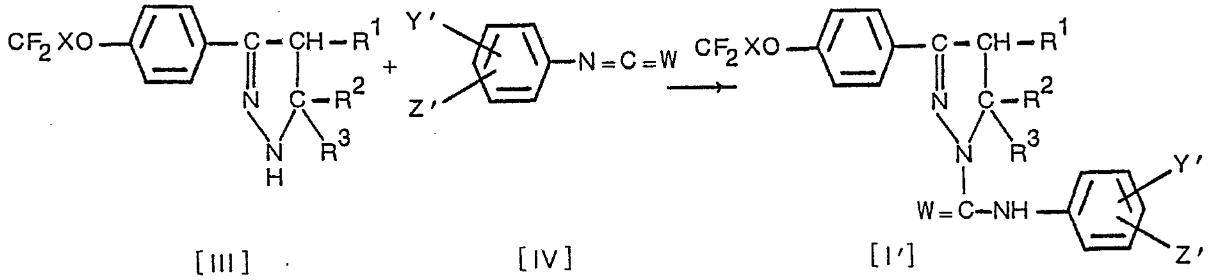

[III] [IV] [I′]

wherein $R^1$ represents hydrogen atom, a $C_{1-4}$ alkyl group, phenyl group, a halogen-substituted phenyl group, a cyano alkyl group or a $C_{1-4}$ alkoxy alkyl group; $R^2$ and $R^3$ respectively represent hydrogen atom, a $C_{1-4}$ alkyl group, phenyl group or a halogen-substituted phenyl group; X represents hydrogen atom or a halogen atom; W represents oxygen or sulphur atom; Y′ and Z′ respectively represent hydrogen atom, a halogen atom, a $C_{1-4}$ alkyl group, a $C_{1-4}$ alkoxy group, nitro-group, trifluoromethyl group, a $C_{1-4}$ alkylthio group, an acetyl group, nitrile group, a $C_{1-4}$ alkyl sufonyl group, a $C_{1-4}$ alkoxycarbonyl group, —A′—$R^5$; or Y′ and Z′ form

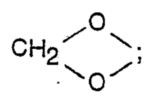

A′ represents —O— or —S— and $R^5$ represents a halogen-substituted $C_{1-4}$ alkyl group.

Scheme 2:

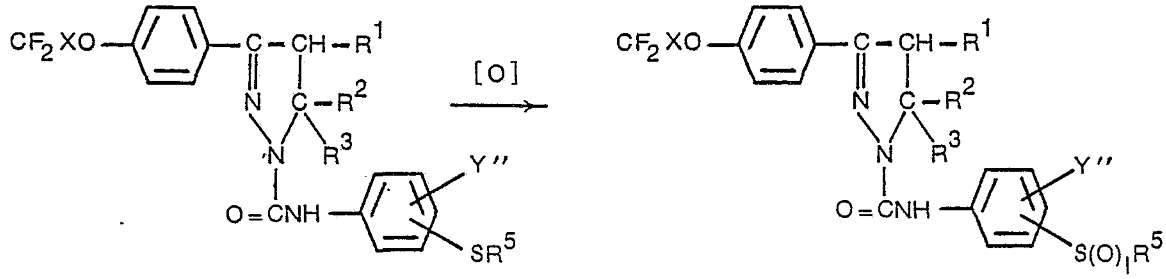


[I″] [I‴]

wherein $R^1$ represents hydrogen atom, a $C_{1-4}$ alkyl group, phenyl group, a halogen-substituted phenyl group, a cyano alkyl group or a $C_{1-4}$ alkoxy alkyl group; $R^2$ and $R^3$ respectively represent hydrogen atom, a $C_{1-4}$ alkyl group, phenyl group or a halogen-substituted phenyl group; X represents hydrogen atom or a halogen atom; Y″ represent hydrogen atom, a halogen atom, a $C_{1-4}$ alkyl group, a $C_{1-4}$ alkoxy group, nitro-group, trifluoromethyl group, an acetyl group, nitrile group, a $C_{1-4}$ alkyl sufonyl group or a $C_{1-4}$ alkoxy-carbonyl group.

The pyrazoline derivatives having the formula (I′) can be produced by reacting 2-pyrazoline derivative having the formula (III) with phenylisocyanate or phenylisothiocyanate having the formula (IV) in the presence or absence of an inert solvent.

Suitable inert solvents include ethyl ether, benzene, toluene, acetonitrile, pyridine, dichloromethane, chloroform and carbon tetrachloride.

The reaction temperature and the reaction time can be selected depending upon the starting material. Usually, the reaction temperature is in a range of −20°C to 100°C. The reaction time is preferably in a range of 0.5 to 24 hours.

The compounds having the formula (I) wherein A is —SO— or —$SO_2$—, can be obtained by oxidizing the compound having the formula [I''] obtained by the Scheme (1) with an oxidizing agent such as hydrogen peroxide-acetic acid or meta chloroperbenzoic acid.

The 2-pyrazoline derivatives having the formula (III) as the starting material used in the reaction scheme are also novel compounds which can be usually produced by the following reaction schemes.

Scheme (3-A):

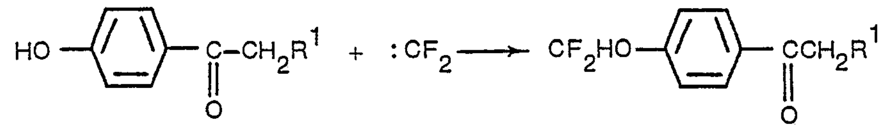

[V] [VI']

wherein $R^1$ represents hydrogen atom, a $C_{1-4}$ alkyl group, or phenyl group, halogen-substituted phenyl group, a cyano alkyl group or $C_{1-4}$ alkoxy alkyl group.

Scheme (3-B):

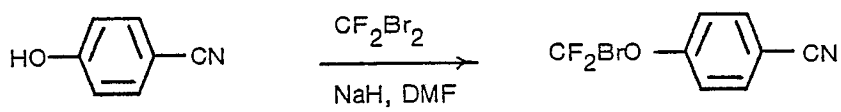


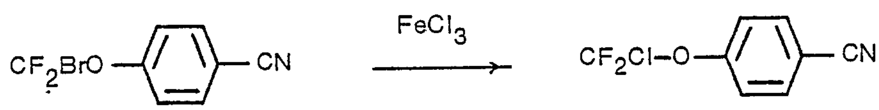


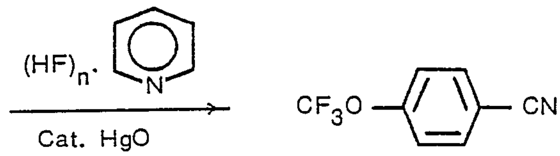


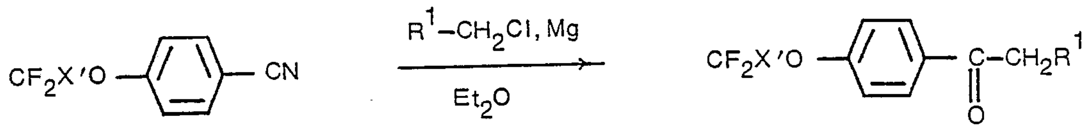


[VI'']

wherein $R^1$ represents hydrogen atom, a $C_{1-4}$ alkyl group, or phenyl group, halogen-substituted phenyl group, or $C_{1-4}$ alkoxy alkyl group; X' represents a halogen atom.

Scheme (4—A):

The compound having the formula (III) wherein $R^1$ represents phenyl group or halogen-substituted phenyl group and $R^2$ and $R^3$ represent hydrogen atom;

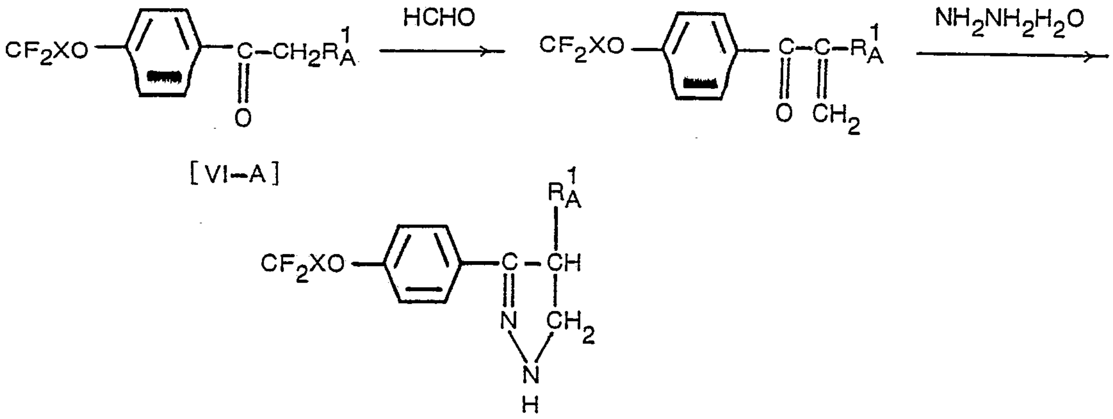


[VI—A]

[III—A]

wherein $R_A^1$ represents phenyl group or halogen-substituted phenyl group; X represents a hydrogen atom or a halogen atom.

Scheme (4—B):

The compound having the formula (III) wherein $R^1$ represents hydrogen atom, a $C_{1-4}$ alkyl group, a cyano alkyl group or a $C_{1-4}$ alkoxy alkyl group; $R^2$ and $R^3$ represent hydrogen atom.

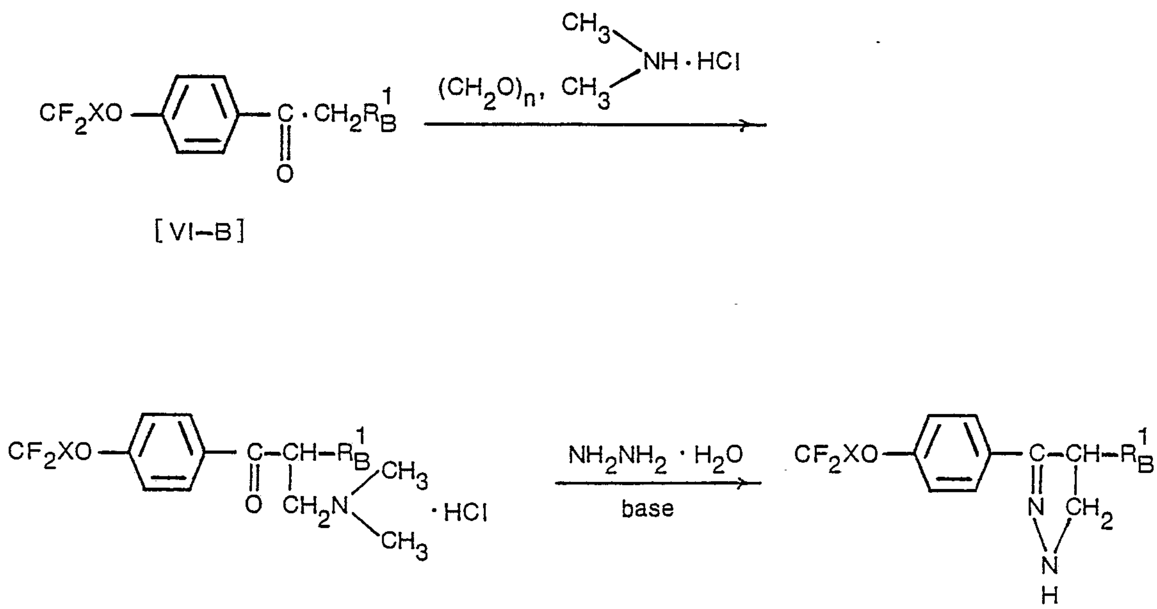


[VI—B]

[III—B]

wherein $R_B^1$ represents hydrogen atom, a $C_{1-4}$ alkyl group; a cyano alkyl group or a $C_{1-4}$ alkoxy group; X represents a hydrogen atom or a halogen atom.

Scheme (4—C):

The compound having the formula (III) wherein $R^2$ represents a $C_{1-4}$ alkyl group, phenyl group, or a halogen-substituted phenyl group; $R^3$ represents a hydrogen atom.

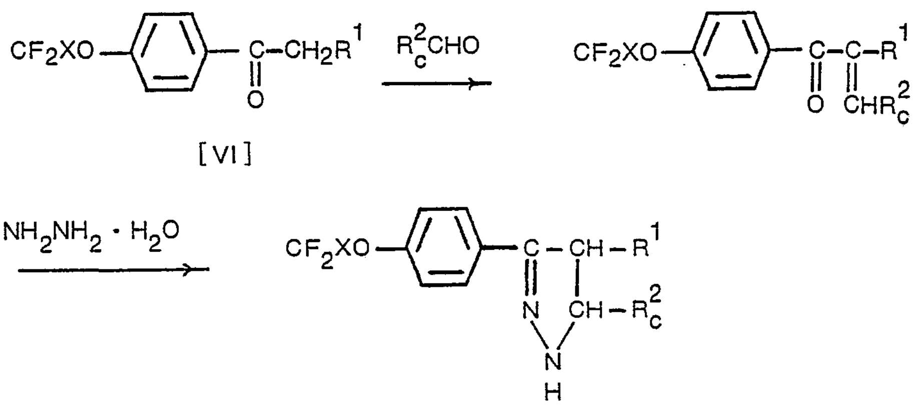


[VI]

[III—C]

wherein $R^1$ represents hydrogen atom, a $C_{1-4}$ alkyl group, phenyl group, a halogen-substituted phenyl group, a cyano alkyl group or a $C_{1-4}$ alkoxy alkyl group and $R^2_c$ represents a $C_{1-4}$ alkyl group, phenyl group or halogen-substituted phenyl group; X represents a hydrogen atom or a halogen atom.

Scheme (4—D):

The compounds having the formula (III) wherein $R^2$ and $R^3$ respectively represent a $C_{1-4}$ alkyl group, phenyl group or a halogen-substituted phenyl group.

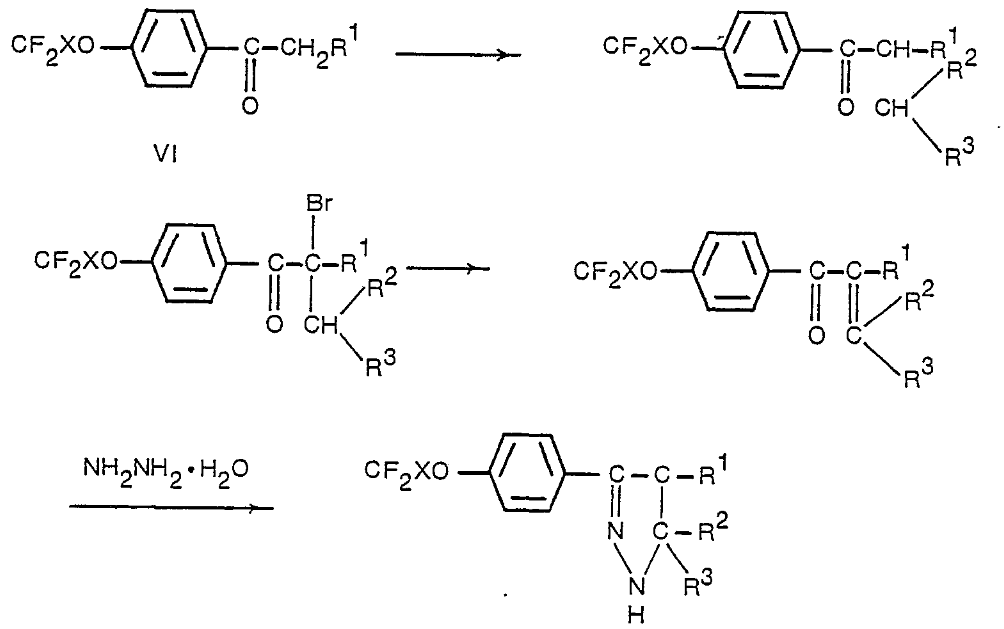


VI

[III—D]

wherein $R^1$ represents hydrogen atom, a $C_{1-4}$ alkyl group, phenyl group or a halogen-substituted phenyl group, a cyano alkyl group or a $C_{1-4}$ alkoxy alkyl group, and $R^2$ and $R^3$ respectively represent a $C_{1-4}$ alkyl group, phenyl group or halogen-substituted phenyl group; X represents a hydrogen atom or a halogen atom.

The 4-halomethoxy acyl benzene having the formula (VI') or (VI'') is produced by selecting from the schemes (3—A) and (3—B) depending upon the X.

The 2-pyrazoline derivatives having the formula (III) can be produced by selecting from the schemes (4—A), (4—B), (4—C) and (4—D) depending upon the substituent groups of $R^1$, $R^2$ and $R^3$ in the formula (III).

In the scheme (4—A), the 2-pyrazoline derivative having the formula (III—A) is produced by reacting formaldehyde in an acidic medium in the presence of a solvent and a catalyst and reacting the product with hydrazine in a solvent such as an alcohol such as ethanol and propanol.

In the scheme (4—B), the dimethyl aminomethylated product is produced by reacting dimethylamine hydrochloride with paraformaldehyde in a solvent such as ethanol in the presence of an acid catalyst such as conc. hydrochloric acid and then, hydrazine is added to the product in a solvent such as methanol in the presence of a base such as 50% NaOH aqueous solution and the mixture is heated to obtain 2-pyrazoline derivative having the formula (III—B).

In the scheme (4—C), the 2-pyrazoline derivative having the formula (III—C) is produced by reacting an aldehyde derivative in a solvent such as an alcohol such as ethanol in the presence of a base such as NaOH aqueous solution and then reacting the resulting chalcone derivative with hydrazine in an alcohol such as ethanol while heating.

In Scheme (4—D), the acrylophenone derivative is produced by reacting the iodide in the presence of a base such as NaH followed by the reaction of bromine and then, reacting a dehydrogen bromide agent such as LiCl. The 2-pyrazoline derivative having the formula (III—D) is produced by reacting the resulting product with hydrazine at room temperature in the presence of a base such as 50% NaOH solution with a solvent such as methanol etc.

The 2-pyrazoline derivatives having the formula (III) produced by these reactions can be isolated and purified. In many cases, however, these products are unstable and decompose when maintained at room temperature. Thus, certain products should be maintained in nitrogen atmosphere at a low temperature. In a practical operation, the pyrazoline derivative having the formula (I) of the present invention can be produced without an isolation and purification of the 2-pyrazoline derivative (III) by reacting the product with a phenyl isocyanate derivative or a phenyl isothiocyanate derivative.

The compounds having $OCF_2Br$ or $SCF_2Br$ group as A—$R^5$ in the phenylisocyanate derivative having the formula (IV), are novel compounds. The compounds can be produced by the following Scheme.

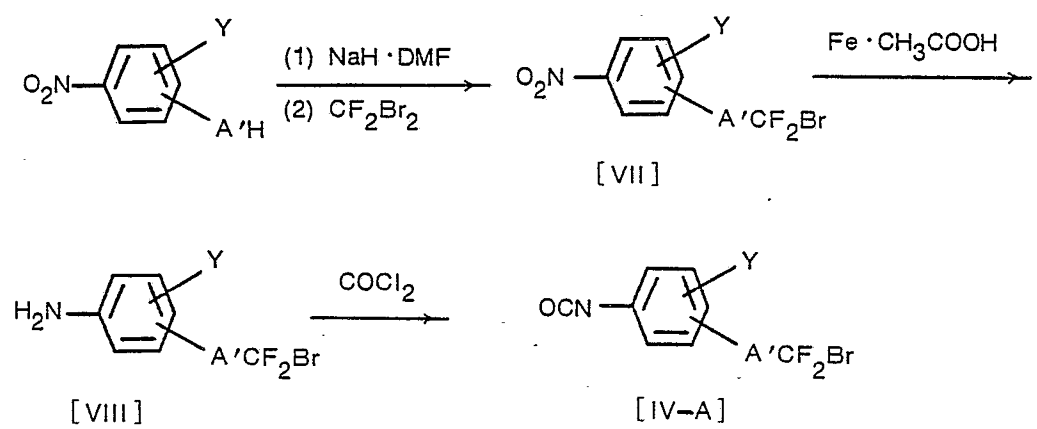


[VII]

[VIII]

[IV—A]

wherein Y and A′ are defined above.

The compound having the formula (VII) is easily produced by reacting NaH with nitrothiophenol or nitrophenol followed by the reaction of dibromodifluoromethane. The resulting product is reduced with iron powder and acetic acid to produce the aniline derivative having the formula (VIII). The isocyanate having the formula (IV—A) is produced by reacting phosgen with the product.

The typical pyrazoline derivatives of the present invention will be described in Tables 1, 2, 3 and 4.

TABLE 1

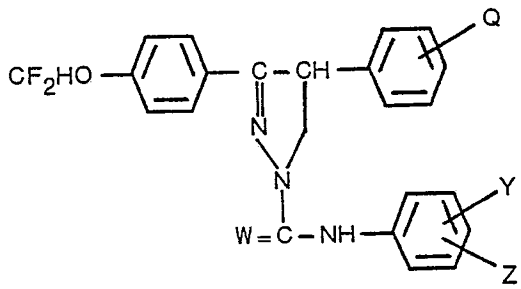

| No. | Q | W | Y, Z | Melting point (°C) |
|---|---|---|---|---|
| 1 | H | O | H | 150 – 152 |
| 2 | H | O | 2–F | |
| 3 | H | O | 3–F | |
| 4 | H | O | 4–F | 163 – 165 |
| 5 | H | O | 2–Cl | 180 – 182 |
| 6 | H | O | 3–Cl | 130 – 132 |
| 7 | H | O | 4–Cl | 147 – 148 |
| 8 | H | O | 3–Br | |
| 9 | H | O | 4–Br | 148 – 152 |
| 10 | H | O | 4–I | 149.5 – 151.5 |
| 11 | H | O | 2,4–$F_2$ | |
| 12 | H | O | 2–F, 4–Cl | 135 – 139 |
| 13 | H | O | 3–Cl, 4–F | 133 – 134.5 |
| 14 | H | O | 2,4–$Cl_2$ | 144 – 147 |
| 15 | H | O | 2,5–$Cl_2$ | |
| 16 | H | O | 3,4–$Cl_2$ | 126 – 128 |
| 17 | H | O | 3,5–$Cl_2$ | 170 – 173 |
| 18 | H | O | 2,4–$Br_2$ | |
| 19 | H | O | 2–Cl, 6–$CH_3$ | |
| 20 | H | O | 2–$CH_3$, 3–Cl | 158.5 – 160 |
| 21 | H | O | 2–$CH_3$, 4–Cl | 154 – 158 |
| 22 | H | O | 3–Cl, 4–$CH_3$ | 103 – 120 |

TABLE 1 (Continued)

| No. | Q | W | Y, Z | Melting point (°C) |
|---|---|---|---|---|
| 23 | H | O | 2–$CH_3$, 4–Br | |
| 24 | H | O | 3–$CH_3$ | |
| 25 | H | O | 4–$CH_3$ | 145 – 147 |
| 26 | H | O | 2–$C_2H_5$ | |
| 27 | H | O | 4–$C_2H_5$ | |
| 28 | H | O | 4–$CH(CH_3)_2$ | 138.5 – 141 |
| 29 | H | O | 4–n–$C_4H_9$ | |
| 30 | H | O | 3,4–$(CH_3)_2$ | |
| 31 | H | O | 3,5–$(CH_3)_2$ | |
| 32 | H | O | 2–$CH_3$, 6–$C_2H_5$ | |
| 33 | H | O | 2,6–$(C_2H_5)_2$ | |
| 34 | H | O | 2–$OCH_3$, 4–Cl | |
| 35 | H | O | 2–$OCH_3$, 5–Cl | |
| 36 | H | O | 2–$CH_3$, 4–$OCH_3$ | |
| 37 | H | O | 2–$OCH_3$ | |
| 38 | H | O | 4–$OCH_3$ | 142 – 146 |
| 39 | H | O | 4–$OC_2H_5$ | |
| 40 | H | O | 4–O–n$C_4H_9$ | 143 – 145 |
| 41 | H | O | 3,4 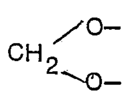 | 98 – 107 |
| 42 | H | O | 3,5–$(OCH_3)_2$ | |
| 43 | H | O | 3–$NO_2$ | |
| 44 | H | O | 4–$NO_2$ | 188 – 193 |
| 45 | H | O | 2–$NO_2$, 4–Cl | |
| 46 | H | O | 3–$NO_2$, 4–F | |

TABLE 1 (Continued)

| No. | Q | W | Y, Z | Melting point (°C) |
|---|---|---|---|---|
| 47 | H | O | 3–$NO_2$, 4–$CH_3$ | |
| 48 | H | O | 2–$CH_3$, 4–$NO_2$ | |
| 49 | H | O | 2–F, 5–$NO_2$ | |
| 50 | H | O | 2–$CH_3$, 5–$NO_2$ | |
| 51 | H | O | 2–$OCH_3$, 5–$NO_2$ | |
| 52 | H | O | 2–$CF_3$ | |
| 53 | H | O | 3–$CF_3$ | 120 – 122 |
| 54 | H | O | 4–$CF_3$ | 163 – 165 |
| 55 | H | O | 2–$CF_3$, 4–Br | |
| 56 | H | O | 3–$CF_3$, 4–Cl | 137 – 140 |
| 57 | H | O | 3,5–$(CF_3)_2$ | |
| 58 | H | O | 3–CN | |
| 59 | H | O | 4–CN | |
| 60 | H | O | 4–$COOC_2H_5$ | 154 – 156 |
| 61 | H | O | 4–$SCH_3$ | 158.5 – 160.5 |
| 62 | H | O | 4–$SO_2CH_3$ | 172.5 – 174 |
| 63 | H | O | 4–$COCH_3$ | 196 – 200 |
| 64 | H | O | 4–$SCF_3$ | 166 – 168 |
| 65 | H | O | 4–$SCF_2Cl$ | 149 – 151 |
| 66 | H | O | 4–$SCF_2Br$ | 133 – 136 |
| 67 | H | O | 4–$SCF_2H$ | |
| 68 | H | O | 4–$SOCF_3$ | 147 – 157 |
| 69 | H | O | 4–$SOCF_2Cl$ | 140 – 147 |
| 70 | H | O | 4–$SOCF_2Br$ | |
| 71 | H | O | 4–$SO_2CF_3$ | 135 – 138 |

TABLE 1 (Continued)

| No. | Q | W | Y, Z | Melting point (°C) |
|---|---|---|---|---|
| 72 | H | O | 4–$SO_2CF_2Cl$ | 144 – 148 |
| 73 | H | O | 4–$SO_2CF_2Br$ | 159 – 162 |
| 74 | H | O | 4–$OCF_3$ | 125 – 126 |
| 75 | H | O | 4–$OCF_2Br$ | 136 – 138 |
| 76 | H | O | 4–$OCF_2Cl$ | |
| 77 | H | O | 4–$OCF_2H$ | 120 – 122 |
| 78 | H | O | 3–Cl, 4–$OCF_2H$ | 118.5 – 121 |
| 79 | H | O | 3–$OCF_2H$, 4–Cl | 112 – 117 |
| 80 | H | O | 3–$OCH_2CF_3$ | 154 – 156 |
| 81 | H | O | 4–$OCH_2CF_3$ | 165.5 – 167.5 |
| 82 | H | O | 4–$OCF_2CF_2H$ | 132 – 135 |
| 83 | H | O | 4–$OCF_2CFHCl$ | |
| 84 | Cl | O | 4–Cl | 124 – 128 |
| 85 | Cl | O | 4–Br | 132 – 136 |
| 86 | Cl | O | 4–$CF_3$ | 143 – 147 |
| 87 | Cl | O | 4–$SCF_3$ | 114 – 117 |
| 88 | Cl | O | 4–$SCF_2Cl$ | 112 – 116 |
| 89 | Cl | O | 4–$SO_2CF_2Cl$ | 168 – 171 |
| 90 | Cl | O | 4–$OCF_3$ | 117 – 119 |
| 91 | Cl | O | 4–$OCF_2CF_2H$ | |
| 92 | F | O | 4–Cl | 130 – 133 |
| 93 | F | O | 4–Br | 135 – 139 |
| 94 | F | O | 4–$CF_3$ | 158 – 162 |
| 95 | F | O | 2–F, 4–Cl | |
| 96 | F | O | 3,4–$Cl_2$ | 139.5 – 142 |
| 97 | F | O | 4–$CH_3$ | 112 – 114 |

TABLE 1 (Continued)

| No. | Q | W | Y, Z | Melting point (°C) |
|---|---|---|---|---|
| 98 | F | O | 4–$OCH_3$ | 114 – 119 |
| 99 | F | O | 4–$NO_2$ | 174 – 178 |
| 100 | F | O | 4–$SCF_3$ | 117 – 120 |
| 101 | F | O | 4–$SCF_2Br$ | 115 – 119 |
| 102 | F | O | 4–$SO_2CF_3$ | 119 – 124 |
| 103 | F | O | 4–$OCF_3$ | 132 – 134 |
| 104 | F | O | 4–$OCF_2Br$ | 121.5 – 123.5 |
| 105 | H | S | H | |
| 106 | H | S | 4–Cl | 158 – 161 |
| 107 | H | S | 4–$CF_3$ | |
| 108 | H | S | 4–$NO_2$ | |

TABLE 2

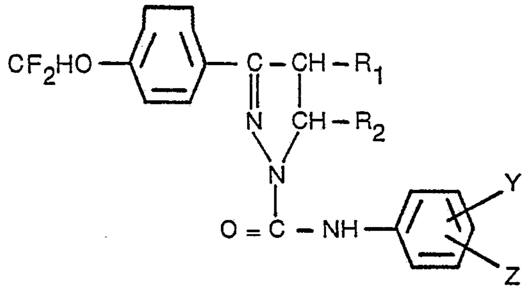

| No. | $R_1$ | $R_2$ | Y, Z | Melting point (°C) |
|---|---|---|---|---|
| 109 | H | H | 3–Cl | 116 – 119 |
| 110 | H | H | 4–Cl | 137 – 140 |
| 111 | H | H | 4–Br | 131 – 134 |
| 112 | H | H | 2–F, 4–Cl | 135 – 136 |
| 113 | H | H | 3,4–$Cl_2$ | 148 – 150 |
| 114 | H | H | 4–$CH_3$ | 122 – 126 |
| 115 | H | H | 4–$OCH_3$ | 110 – 112 |
| 116 | H | H | 3–$CF_3$ | 120 – 122 |
| 117 | H | H | 4–CN | 149 – 154 |
| 118 | H | H | 4–$SCF_3$ | 152.5 – 153.5 |
| 119 | H | H | 4–$SCF_2Cl$ | 139 – 140 |
| 120 | H | H | 4–$OCF_3$ | 100 – 102 |
| 121 | H | H | 4–$OCF_2CF_2H$ | 101 – 103 |
| 122 | $CH_3$ | H | 4–Cl | 96 – 98 |
| 123 | $CH_3$ | H | 4–Br | 67 – 69 |
| 124 | $CH_3$ | H | 4–$CF_3$ | 105 – 108 |
| 125 | $CH_3$ | H | 4–$SCF_3$ | 144 – 146 |
| 126 | $CH_3$ | H | 4–$SCF_2Cl$ | 143 – 146 |
| 127 | $CH_3$ | H | 4–$OCF_3$ | 108 – 111 |
| 128 | $CH_3$ | H | 4–$OCF_2Br$ | 96 – 97 |
| 129 | $CH_3$ | H | 4–$OCF_2CF_2H$ | 124 – 129 |

TABLE 2 (Continued)

| No. | $R_1$ | $R_2$ | Y, Z | Melting point (°C) |
|---|---|---|---|---|
| 130 | $i\text{-}C_3H_7$ | H | $4\text{–}SCF_3$ | 104 – 108 |
| 131 | $i\text{-}C_3H_7$ | H | $4\text{–}SCF_2Cl$ | 57 – 59 |
| 132 | $i\text{-}C_3H_7$ | H | $4\text{–}OCF_3$ | 103 – 107 |
| 133 | $i\text{-}C_3H_7$ | H | $4\text{–}OCF_2CF_2H$ | 86 – 88 |
| 134 | H | 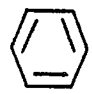 | 4–Cl | 137 – 140 |
| 135 | H | 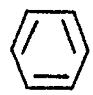 | 4–Br | 145 – 147 |
| 136 | H | 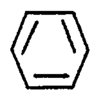 | $4\text{–}NO_2$ | 157 – 158 |
| 137 | H | 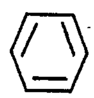 | $4\text{–}CF_3$ | 163 – 165 |
| 138 | H | 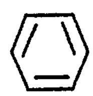 | $4\text{–}CH_3$ | 88 – 96 |
| 139 | H | 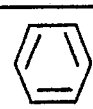 | $4\text{–}OCH_3$ | |
| 140 | H | 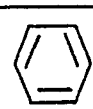 | 4–CN | 157 – 159 |
| 141 | H | 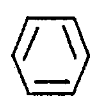 | $3,4\text{–}Cl_2$ | 116 – 120 |
| 142 | H | 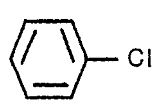 | 4–Br | 167.5–169.5 |
| 143 | H | 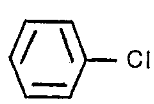 | 4–Cl | 159 – 161 |
| 144 | H | 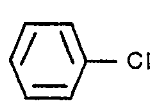 | $4\text{–}CF_3$ | 175 – 177 |

TABLE 2 (Continued)

| No. | $R_1$ | $R_2$ | Y, Z | Melting point (°C) |
|---|---|---|---|---|
| 145 | $(CH_2)_3CN$ | H | 4–Cl | 117 – 119 |
| 146 | $(CH_2)_3CN$ | H | 3,4–$Cl_2$ | 120 – 124 |
| 147 | $(CH_2)_3CN$ | H | 4–$CF_3$ | 162 – 164 |
| 148 | $(CH_2)_3CN$ | H | 4–$SCF_3$ | 137.5 – 139 |
| 149 | $(CH_2)_3CN$ | H | 4–$SO_2CF_3$ | 166 – 168 |
| 150 | $(CH_2)_2OCH_3$ | H | 4–$SCF_2Cl$ | |
| 151 | $(CH_2)_2OCH_3$ | H | 4–$OCF_2CF_2H$ | |

TABLE 3

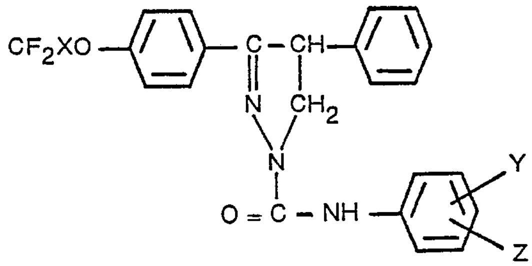

| No. | X | Y, Z | Melting point (°C) |
|---|---|---|---|
| 152 | F | 4–Cl | 144 – 150 |
| 153 | F | 4–I | 139 – 142 |
| 154 | F | 3,4–$Cl_2$ | 112 – 114 |
| 155 | F | 4–$CF_3$ | 125 – 128 |
| 156 | F | 4–$OCF_3$ | 141 – 143.5 |
| 157 | F | 4–$OCF_2CF_2H$ | 131 – 134 |
| 158 | F | 4–$SCF_3$ | 170 – 171 |
| 159 | F | 4–$SCF_2Cl$ | 141 – 144 |
| 160 | Br | 4–Cl | 132 – 134.5 |
| 161 | Br | 3,4–$Cl_2$ | 120 – 124 |
| 162 | Br | 4–$OCF_3$ | 131 – 135 |
| 163 | Br | 4–$OCF_2CF_2H$ | 142.5 – 145 |
| 164 | Br | 4–$SCF_3$ | 150 – 152.5 |
| 165 | Br | 4–$SCF_2Cl$ | 148 – 150 |
| 166 | Br | 4–$CF_3$ | 152.5 – 154 |

TABLE 4

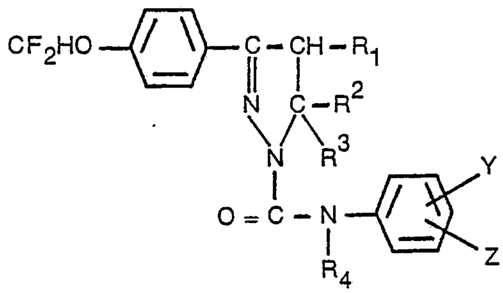

| No. | $R_1$ | $R_2$ | $R_3$ | $R_4$ | Y, Z | Melting point (°C) |
|---|---|---|---|---|---|---|
| 167 | 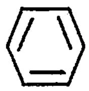 | H | H | $CH_3$ | 4–Cl | 103 – 107 |
| 168 | 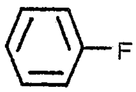 | H | H | $CH_3$ | 4–$CF_3$ | |
| 169 | 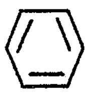 | H | H | $nC_3H_7$ | 4–$OCF_2CF_2H$ | |
| 170 | 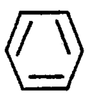 | H | H | $CH_3$ | 4–$SCF_3$ | |
| 171 | 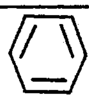 | $CH_3$ | $CH_3$ | H | 4–Cl | 112 – 113.5 |
| 172 | 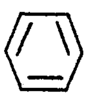 | $CH_3$ | $CH_3$ | H | 3,4–$Cl_2$ | 94 – 97 |
| 173 | 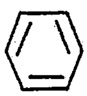 | $CH_3$ | $CH_3$ | H | 4–$CF_3$ | 153 – 157 |
| 174 | 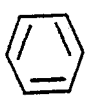 | $CH_3$ | $CH_3$ | H | 4–$SCF_3$ | 139.5 – 141 |
| 175 | 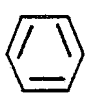 | $CH_3$ | $CH_3$ | H | 4–$OCF_3$ | 57 – 60 |
| 176 | 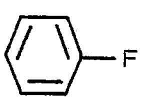 | $CH_3$ | $CH_3$ | H | 4–$OCF_2H$ | |

Certain compounds of the present invention include geometrical isomers and optical isomers having asymmetric carbon atom at 4- or 5-position of 2-pyrazoline ring. These isomers are also included in the compounds of the present invention.

The serial numbers of the compounds described in Tables 1, 2, 3, & 4 are referred in the following Preparations, Compositions and Tests.

The compounds of the present invention are useful as insecticides for controlling insect pests in sanitation, and various insect pests in agriculture and horticulture which cause damages to rice, vegetable, fruits, cotton, and other crop plants and flowers and insect pests in forest and insect pests in storages.

The typical insect pests which are controlled by the compounds of the present invention are provided for purposes of illustration only.

*Orthoptera*

| | |
|---|---|
| German Cockroach | (*Blattella germanica*) |
| Rice Grasshopper | (*Oxya yezoensis*) |

*Thysanoptera*

| | |
|---|---|
| Rice Thrips | (*Baliothrips biformis*) |

*Hemiptera*

| | |
|---|---|
| Rice Stink Bug | (*Lagynotomus elongatus*) |
| Green Stink Bug | (*Nezcra antennata*) |
| Rice Bug | (*Leptocorisa chinensis*) |
| Bean Bug | (*Riotortus clavatus*) |
| Cotton Bug | (*Dysdercus cingulatus*) |
| Grape Leafhopper | (*Epiacanthus stramineus*) |
| Green Rice Leafhopper | (*Nephotettix cincticeps*) |
| Small Brown Planthopper | (*Laodelphax striatellus*) |
| Brown Rice Planthopper | (*Nilaparvata lugens*) |
| White-backed Rice Planthopper | (*Sogatella furcifera*) |
| Citrus Psylla | (*Diaphorina citri*) |
| Greenhouse Whitefly | (*Trialeurodes vaporariorum*) |
| Cowpea Aphid | (*Aphis craccivora*) |
| Cotton Aphid | (*Aphis gossypii*) |
| Apple Aphid | (*Aphis spiraecola*) |
| Green Peach Aphid | (*Myzue persicae*) |
| Citrus Mealybug | (*Planococcus citri*) |
| Comstock Mealybug | (*Pseudcoccus censtocki*) |
| Red Scale | (*Aonidiella aurantri*) |
| San Jose Scale | (*Comstockaspis perniciosa*) |
| Arrowhead Scale | (*Unaspis yanonensis*) |

*Lepidoptera*

| | |
|---|---|
| Apple Leafminer | (*Phyllonorycfer ringoneella*) |
| Citrus Leafminer | (*Phyllocnistis citrella*) |
| Diamondback Moth | (*Plutella xylostella*) |
| Pink Bollworm | (*Pectinophora gossypiella*) |
| Potato Tuberworm | (*Phthorimaea operculella*) |
| Peach Fruit Moth | (*Carposina niponensis*) |
| Summer Fruit Tortrix | (*Adoxophyes orana*) |
| Oriental Fruit Moth | (*Grapholita molesta*) |
| Soybean Pod Borer | (*Leguminivora glycinivorella*) |
| Rice Stem Borer | (*Chilo suppressalis*) |
| Rice Leafroller | (*Chaphalocrocis medinalis*) |
| Pea Pod Borer | (*Etiella zinckenella*) |
| Oriental Corn Borer | (*Ostrinia furnacalis*) |
| Yellow Rice Borer | (*Tryporyza incertulas*) |
| Cutworm | (*Agrotis segetum*) |
| Cotton Looper | (*Anomis flava*) |
| American Bollworm, Cotton Bollworm or Tobacco Budworm | (*Heliothis armigera, H. zea H. virescens*) |
| Cabbage armyworm | (*Mamestra brassicae*) |
| Beet Semi Looper | (*Plusia nigrisigna*) |
| Rice Armyworm | (*Pseudaletia separata*) |
| Pink Borer | (*Sesamia inferens*) |
| Common Cutworm | (*Spodoptera litura*) |
| Common White | (*Pieris rapae crucivora*) |
| Smaller Citrus Dog | (*Papilio xuthus*) |
| Rice Skipper | (*Parnara guttata*) |
| Codling Moth | (*Cydia pomonella*) |

*Coleoptera*

| | |
|---|---|
| Cupreous Chafer | (*Anomala cuprea*) |
| Asiatic Garden Beetle | (*Maladera castanea*) |
| Japanese Beetle | (*Popillia Japonica*) |
| Twenty-eight-spotted Ladybeetle | (*Henosepilachna vigintioctopunctata*) |
| Cucurbit Leaf Beetle | (*Aulacophora femoralis*) |
| Rice Leaf Beetle | (*Oulema oryzae*) |
| Striped Flea Beetle | (*Phyllotreta striolata*) |
| Rice Plant Weevil | (*Echinocnemus squameus*) |
| Rice Water Weevil | (*Lissorhoptrus oryzophilus*) |
| Vegetable Weevil | (*Listroderes obliquus*) |
| Maize Weevil | (*Sitophilus zeamais*) |
| Bull Weevil | (*Anthonomus grandis*) |
| Corn Rootworms | (*Diabrotica* spp.) |
| Colorado Potato Beetle | (*Leptinotarsa decemlineata*) |

*Hymenoptera*

| | |
|---|---|
| Fire Ant | (*Solenopsis geminata*) |

*Diptera*

| | |
|---|---|
| Soybean Pud Gall Midge | (*Asphondylia* spp.) |
| Oriental Fruit Fly | (*Dacus dorsalis*) |
| Rice Leafminer | (*Hydrellia griseola*) |
| Rice Stem Maggot | (*Chlorops oryzae*) |
| Rice Leafminer | (*Agromyza oryzae*) |
| Seedcorn Maggot | (*Hylemya platura*) |
| Mediterranean Fruit Fly | (*Ceratitis capitata*) |
| Rice Gall Midge | (*Orseolia oryzae*) |
| House Fly | (*Musca domestica*) |
| Pale House Mosquito | (*Culex pipiens pallens*) |

*Isoptera*

| | |
|---|---|
| Termites | (*Coptotermes formosanus*) |

The insecticidal activity of the compounds of the present invention is imparted to not only young larva but also old larva in direct or in penetration by direct contact or immersion. The compounds of the present invention are also effective to control various acarina and nematode.

In the application of the insecticidal composition of the present invention, it is preferable to apply it at a concentration of 0.01 to 10,000 ppm preferably 0.1 to 2,000 ppm of the active ingredient. In order to control aquatic insect pests, the composition having said concentration can be sprayed to the part to control the aquatic insect pests. Therefore, the concentration of the active ingredient in water can be lower.

In the application of the compound of the present invention as the insecticide, it is preferable to prepare a composition by mixing the active ingredient with a desired solid carrier such as clay, talc and bentonite; or a liquid carrier such as water, alcohols (methanol, ethanol etc.), ketones, ethers, aliphatic hydrocarbons, aromatic hydrocarbons (benzene, toluene, xylene etc.), esters and nitriles, if necessary, with an emulsifier, a dispersing agent, a suspending agent, a spreader, a penetrant and a stabilizer so as to form suitable compositions for practical applications in the form of an emulsifiable concentrate, an oil spray, a wettable powder, a dust, a granule, a tablet, a paste, a flowable, a bait poison, an aerosol, a fumigrant, a mosquito-coil and mosquito mat.

It is possible to blend the active ingredient of the present invention to a suitable other active ingredient such as the other insecticides, germicides, herbicides, plant growth regulators, and fertilizers in the preparation of the composition or in the application.

The present invention will be further illustrated by certain examples of Preparations, Compositions and Tests which are provided for purposes of illustration only.

Preparation 1:

1-(4-Chlorophenylcarbamoyl)-3-(4-difluoromethoxyphenyl)-4-phenyl-2-pyrazoline (Compound No. 7)

(a) *Preparation of 3-(4-difluoromethoxyphenyl)-4-phenyl-2-pyrazoline (Intermediate)*

A mixture of 17 g. of benzyl-p-(hydroxy)phenyl ketone and 30 g. of sodium hydroxide in 40 ml. of water and 50 ml. of dioxane was heated at 70 to 80°C and 22 g. of Freon® 22 gas was fed into the solution during 1 hour while heating. After cooling the reaction mixture, 150 ml. of water and 150 ml. of ethyl ether were added to the reaction mixture and an organic phase was obtained by an extraction. The organic phase was separated and dried over anhydrous sodium sulfate and ethyl ether was distilled off to obtain 17.6 g. of benzyl-p-(difluoromethoxy)phenyl ketone (melting point of 39.0—40.0°C). Into a mixture of 0.9 ml. of piperidine, 0.9 ml. of acetic acid, 25 ml of 37% formaline and 180 ml. of methanol, 17.5 g. of the resulting compound was added and the mixture was refluxed for 3 hours to react them. The reaction mixture was concentrated under a reduced pressure and 150 ml. of water and 200 ml. of chloroform were added. The resulting organic phase was separated and dried over anhydrous sodium sulfate and chloroform was distilled off to obtain 18.0 g. of 4′-difluoromethoxy-2-phenylacrylophenone ($N_D^{20}$ 1.5819). A mixture of 17.5 g. of the product, 8 ml. of hydrazine hydrate and 150 ml. of ethanol was refluxed for 3 hours to react them. After the reaction, the reaction mixture was concentrated under a reduced pressure and 80 ml. of water and 100 ml. of chloroform were added. The resulting organic phase was separated and dried over anhydrous sodium sulfate and chloroform was distilled off to obtain 17.5 g. of 3-(4-difluoromethoxyphenyl)-4-phenyl-2-pyrazoline (melting point of 65—75°C).

(b) *Preparation of Compound No. 7*

A mixture of 5.8 g. of 3-(4-difluoromethoxyphenyl)-4-phenyl-2-pyrazoline obtained in the step (a) and 3.1 g. of p-chlorophenyl isocyanate in 200 ml. anhydrous ethyl ether was refluxed for 6 hours to react them. After cooling, the precipitated crystal (5.3 g.) was separated by a filtration.

It was confirmed that the product was 1-(4-chlorphenylcarbamoyl)-3-(4-difluoromethoxyphenyl)-4-phenyl-2-pyrazoline (melting point of 147.0—148.0°C) by the NMR spectrum (I).

Preparation 2:

1-(3,4-Dichlorophenylcarbamoyl)-3-(4-difluoromethoxyphenyl)-4-phenyl-2-pyrazoline (Compound No. 16)

A mixture of 5.8 g. of 3-(4-difluoromethoxyphenyl)-4-phenyl-2-pyrazoline obtained in the step (a) of Preparation 1, and 3.8 g. of 3,4-dichlorophenyl isocyanate in 200 ml. of anhydrous ethyl ether was refluxed for 6 hours to react them. After cooling the reaction mixture, the precipitated crystal (5.3 g.) was separated by a filtration. It was confirmed that the product was 1-(3,4-dichlorophenylcarbamoyl)-3-(4-difluoromethoxyphenyl)-4-phenyl-2-pyrazoline (melting point of 126.0—128.0°C) by the NMR spectrum (II).

Preparation 3:

1-(4-Chlorophenylcarbamoyl)-3-(4-difluoromethoxyphenyl)-4-phenyl-2-pyrazoline (Compound No. 106)

A mixture of 5.8 g. of 3-(4-difluoromethoxyphenyl)-4-phenyl-2-pyrazoline and 3.4 g. of p-chlorophenylisothiocyanate in 200 ml. of anhydrous ethyl ether was refluxed for 6 hours to react them. After cooling the reaction mixture, the precipitated crystal (6.1 g.) was separated by a filtration. It was confirmed that the product was 1-(4-chlorophenylthiocarbamoyl)-3-(4-difluoromethoxyphenyl)-4-phenyl-2-pyrazoline (melting point of 158.0—161.0°C) by the NMR spectrum (III).

Preparation 4:

1-(4-Trifluoromethylphenylcarbamoyl)-3-(4-difluoromethoxyphenyl)-4-(4-chlorophenyl)-2-pyrazoline (Compound No. 86)

(a) *Preparation of 3-(4-difluoromethoxyphenyl)-4-(4-chlorophenyl-2-pyrazoline (Intermediate)*

In accordance with the process of the step (a) of Preparation 1, 4-chlorobenzyl-4'-(difluoromethoxy)phenyl ketone (melting point of 74.0—76.0°C) was produced as an intermediate by using 20 g. of 4-chlorobenzyl-4'-(hydroxy)phenyl ketone instead of benzyl-p-(hydroxy)phenyl ketone. Then, 4'-difluoromethoxy-2-(4-chlorophenyl) acrylophenone($N_D^{20.5}$ 1.5752) was obtained by reacting the intermediate with formaline. Then, a reaction of hydrazine hydrate with the product was carried out to obtain 11.6 g. of 3-(4-difluoromethoxyphenyl)-4-(4-chlorophenyl)-2-pyrazoline. The product was used as the intermediate for the next step without a purification.

(b) *Preparation of Compound No. 86*

Into 150 ml. of anhydrous ethyl ether, 6.5 g. of 3-(4-difluoromethoxyphenyl)-4-(4-chlorophenyl)-2-pyrazoline obtained in the step (a) and 3.7 g. of p-trifluoromethylphenylisocyanate were added and the mixture was kept at room temperature for one night and the precipitated crystal (6.5 g.) was separated by a filtration. It was confirmed that the product was 1-(4-trifluoromethylphenylcarbamoyl)-3-(4-difluoromethoxyphenyl)-4-(4-chlorophenyl)-2-pyrazoline (melting point of 143.0—147.0°C) by the NMR spectrum (IV).

Preparation 5:

1-(4-Methoxyphenylcarbamoyl)-3-(4-difluoromethoxyphenyl)-2-pyrazoline (Compound No. 115)

(a) *Preparation of 3-(4-difluoromethoxyphenyl)-2-pyrazoline (Intermediate)*

In a reactor A, 54.4 g. of p-hydroxyacetophenone was added to a solution of 17 g. of sodium hydroxide in 160 ml. of water and 400 ml. of dioxane.

On the other hand, in a reactor B, a solution of 80 g. of sodium hydroxide in 300 ml. of water and 360 ml.
of dioxane was prepared. The reactor B was heated to 80°C and Freon® 22 gas was fed and the resulting difluorocarbene was fed through a polytetrafluoroethylene tube into the reactor A at room temperature. After feeding 200 g. of Freon® 22 gas, the reactor A was cooled and 400 ml. of water and 500 ml. of ethyl ether were charged for an extraction. The resulting organic phase was separated and dried over anhydrous sodium sulfate and ethyl ether was distilled off to obtain a crude product. The crude product was distilled under a reduced pressure to obtain 44.6 g. of p-difluoromethoxyacetophenone having a boiling point of 98—100°C 4 mbar (3 mmHg). A mixture of 44 g. of the resulting product, 19.3 g. of dimethylamine hydrochloride, 7.3 g. of paraformaldehyde and 30 ml. of ethanol and 3 ml. of conc. hydrochloric acid was refluxed for 3 hours to react them. The solvent was distilled off under a reduced pressure and the crystal as the residue was obtained. The residue was mixed with 18 ml. of acetone and the crystal was separated by a filtration to obtain 4'-difluoromethoxy-3-dimethylaminopropiophenone hydrochloride having a melting point of 135.0—140.0°C.

A mixture of the resulting crystal 150 ml. of methanol, 33 ml of hydrazine hydrate, 17 ml. of 50% NaOH aq. sol. and 45 ml. of water was refluxed for 1 hour. Methanol was distilled off and then, dichloromethane and water were added for an extraction. The resulting organic phase was separated and dried over anhydrous sodium sulfate and dichloromethane was distilled off to obtain 32 g. of 3-(4-difluoromethoxyphenyl)-2-pyrazoline. The product was used as the intermediate in the next reaction step without any purification.

(b) *Preparation of Compound No. 115*

Into 150 ml of anhydrous ethyl ether, 4.2 g. of 3-(4-difluoromethoxyphenyl)-2-pyrazoline obtained in the step (a) and 3.0 g. of p-methoxyphenylisocyanate were charged and the mixture was kept at room temperature for one night and the precipitated crystal (5.2 g.) was separated by a filtration.

It was confirmed that the product was 1-(4-methoxyphenylcarbamoyl)-3-(4-difluoromethoxyphenyl)-2-pyrazoline (melting point of 110.0—112.0°C) by the NMR spectrum (V).

Preparation 6:

1-(4-Chlorophenylcarbamoyl)-3-(4-difluoromethoxyphenyl)-4-methyl-2-pyrazoline (Compound No. 122)

(a) *Preparation of 3-(4-difluoromethoxyphenyl)-4-methyl-2-pyrazoline (Intermediate)*

In accordance with the process of Preparation 5, p-difluoromethoxy propiophenone (boiling point of 108—109°C 4 mbar 3 mmHg) was produced as an intermediate by using 60 g. of p-hydroxypropiophenone instead of p-hydroxyacetophenone. Then, the product was dimethylaminomethylated to obtain 4'-difluoromethoxy-3-dimethylamino-2-methylpropiophenone hydrochloride (melting point of 134.0—136.0°C). Then, a reaction of hydrazine hydrate with the product in the presence of 50% NaOH aq. sol. was carried out to obtain 37 g. of 3-(4-difluoromethoxyphenyl)-4-methyl-2-pyrazoline. The product was used as the intermediate for the next step without a purification.

(b) *Preparation of Compound No. 122*

Into 150 ml. of anhydrous ethyl ether, 4.7 g. of 3-(4-difluoromethoxyphenyl)-4-methyl-2-pyrazoline obtained in the step (a) and 3.1 g. of p-chlorophenylisocyanate were added and the mixture was kept at room temperature for one night and then ethyl ether was distilled off under a reduced pressure to obtain a crude product. The crude product was purified by a silica gel thin layer chromatography (developing solvent: benzene: ethyl acetate = 5:1) to obtain 3.1 g. of the product in the crystalline form.

In was confirmed that the product was 1-(4-chlorophenylcarbamoyl)-3-(4-difluoromethoxyphenyl)-4-methyl-2-pyrazoline (melting point of 96.0—98.0°C) by the NMR spectrum (VI).

Preparation 7:

1-(4-Bromophenylcarbamoyl)-3-(4-difluoromethoxyphenyl)-5-(4-chlorophenyl)-2-pyrazoline (Compound No. 142)

(a) *Preparation of 3-(4-difluoromethoxyphenyl)-5-(4-chlorophenyl)-2-pyrazoline (Intermediate)*

Into 10 ml. of ethanol and 20 ml. of 10% NaOH aqueous solution, 5.6 g. of p-difluoromethoxyacetophenone obtained as the intermediate of step (a) of preparation 5 was charged and 4.2 g. of p-chlorobenzaldehyde was added to the mixture at room temperature while stirring and the mixture was stirred for 1 hour. The crystal was precipitated. The mixture was cooled to 0°C and kept at 0°C for 1 hour. The precipitated crystal was separated by a filtration and washed twice with 20 ml. of water and once with 20 ml. cold ethanol and dried under a reduced pressure for 3 hours to obtain 8.4 g. of 4-chloro-4'-difluoromethoxy chalcone (melting point of 114—115°C).

A mixture of 8.4 g. of the product in 6 ml. of hydrazine hydrate and 80 ml. of ethanol was refluxed for 2 hours while heating. After the reaction, the reaction mixture was concentrated under a reduced pressure and then, 50 ml. of water and 80 ml. of chloroform were added. The organic phase was separated and dried over anhydrous sodium sulfate and chloroform was distilled off to obtain 8.8 g. of 3-(4-difluoromethoxyphenyl)-5-(4-chlorophenyl)-2-pyrazoline.

(b) *Preparation of Compound No. 142*

Into 20 ml. of anhydrous ethyl ether, 1 g. of 3-(4-difluoromethoxyphenyl)-5-(4-chlorophenyl)-2-pyrazoline obtained in the step (a) and 0.5 g. of p-bromophenylisocyanate were charged to react them at room temperature for 15 hours. The precipitated crystal was separated by a filtration.

It was confirmed that the product was 1-(4-bromophenylcarbamoyl)-3-(difluoromethoxyphenyl)-5-(4-chlorophenyl)-2-pyrazoline (melting point of 167.5—169.5°C) by the NMR spectrum (VII).

Preparation 8:

1-(4-Trifluoromethylthiophenylcarbamoyl)-3-(4-difluoromethoxyphenyl)-4-phenyl-2-pyrazoline

*Preparation of Compound No. 64*

Into 20 ml. of anhydrous ethyl ether 5.8 g of 3-(4-difluoromethoxyphenyl)-4-phenyl-2-pyrazoline and 4.4 g. of p-trifluoromethylthiophenylisocyanate were charged. The mixture was refluxed for 6 hours and cooled. The precipitated crystal was separated by a filtration. The yield was 6.2 g.

It was confirmed that the product was 1-(4-trifluoromethylthiophenylcarbamoyl)-3-(4-difluoromethoxyphenyl)-4-phenyl-2-pyrazoline(melting point of 166—168°C) by the NMR spectrum (VIII).

Preparation 9:

1-(4-Trifluoromethylsulfinylphenylcarbamoyl-3-(4-difluoromethoxyphenyl)-4-phenyl-2-pyrazoline

*Preparation of Compound No. 68*

Into 20 ml. of acetic acid, 0.74 g. of 1-(4-trifluoromethylthiophenylcarbamoyl)-3-(4-difluoromethoxyphenyl)-4-phenyl-2-pyrazoline obtained by the process of Preparation 8 was charged and 0.2 ml. of 30% $H_2O_2$ was charged. The mixture was stirred for 24 hours at room temperature and then, 0.2 ml. of 30% $H_2O_2$ was further charged and the mixture was stirred for 72 hours. Into the reaction mixture, 60 ml. of water was added and the product was extracted two times with 60 ml. of water was added and the product was extracted two times with 60 ml. of chloroform. The chloroform layer was dried over anhydrous magnesium sulfate. Chloroform was distilled off under a reduced pressure. The residue was admixed with 40 ml. of n-hexane-ethyl ether (1:1) and the precipitated crystal was separated by a filtration. The yield was 0.5 g.

It was confirmed that the product was 1-(4-trifluoromethylsulfinylphenylcarbamoyl)-3-(4-difluoromethoxyphenyl)-4-phenyl-2-pyrazoline (melting point of 147—157°C) by the NMR spectrum (IX).

Preparation 10:

1-(4-Bromodifluoromethylthiophenylcarbomoyl)-3-(4-difluoromethoxyphenyl)-4-phenyl-2-pyrazoline

*Preparation of Compound No. 66*

(a) *Preparation of p-bromodifluoromethylthiophenylisocyanate*

On an ice bath, NaH (50% 8.0 g. 167 m mol) was suspended in dimethylformamide (150 ml.) and a solution of p-nitrothiophenol (25 g. 161 m mol) in dimethylformamide (200 ml.) was added and the mixture was stirred for 30 minutes on the ice bath. A solution of dibromodifluoromethane (50 g. 238 m mol) in dimethylformamide (100 ml.) was added and the mixture was stirred for 1 hour on the ice bath and further stirred for 5 hours at room temperature. Water was added to the reaction mixture and the product was

extracted with methylene chloride, and dried over anhydrous magnesium sulfate. A crude product obtained by concentrating the extract, was purified by a column chromatography to obtain 40.4 g. of p-(bromodifluoromethylthio)nitrobenzene (melting point of 72—73°C). The yield was 88%.

Into ethanol (300 ml.), the resulting p-(bromodifluoromethylthio)nitrobenzene (32.4 g.), iron powder (25 g.) and acetic acid (60 ml.) were added. The mixture was refluxed for 1 hour. After removing most of the solvent under a reduced pressure, water was added and the product was extracted with ether. The extract was washed with NaCl aq. sol. and dried over anhydrous magnesium sulfate. After removing the solvent, the product was purified by a column chromatography to obtain 15.5 g. of p-(bromodifluoromethylthio) aniline. The yield was 54%.

A solution of p-(bromodifluoromethylthio) aniline (4 g.) in 40 ml. of ethyl acetate was added dropwise while feeding phosgen. After the addition, phosgen was further fed for 10 minutes. The reaction mixture was concentrated under a reduced pressure to obtain 4 g. of the product ($N_D^{20}$ 1.5790). The product was used without any purification as a reagent in the next reaction.

(b) *Preparation of Compound No. 66*

Into 50 ml. of anhydrous ethyl ether, 5.6 g. of p-bromodifluoromethylthiophenylisocyanate obtained in the step (a) and 5.8 g. of 3-(4-difluoromethoxyphenyl)-4-phenyl-2-pyrazoline obtained in the step (a) of Preparation 1 were charged and the mixture was stirred for 6 hours at room temperature. The precipitated crystal was separated by a filtration and washed with n-hexane. The yield was 5.8 g.

It was confirmed that the product was 1-(4-bromodifluoromethylthiophenylcarbamoyl)-3-(4-difluoromethoxyphenyl)-4-phenyl-2-pyrazoline (melting point of 133—136°C) by the NMR spectrum (X).

Preparation 11:

1-(4-Bromodifluoromethylsulfonylphenylcarbamoyl)-3-(4-difluoromethoxyphenyl)-4-phenyl-2-pyrazoline

*Preparation of Compound No. 73*

Into 40 ml. of acetic acid, 4 g. of 1-(4-bromodifluoromethylthiophenylcarbamoyl)-3-(4-difluoromethoxyphenyl)-4-phenyl-2-pyrazoline was dissolved and 80 ml. of 30% $H_2O_2$ was added and the mixture was heated at 60°C. After 4 hours, water was added to the reaction mixture. The product was extracted two times with 50 ml. of chloroform. The chloroform layer was washed with 50 ml. of $NaHCO_3$ aq. sol. and dried over anhydrous sodium sulfate. The solvent was distilled off under a reduced pressure to obtain 3.8 g. of the crude product. The product was recrystallized by using 20 ml. of n-hexane-ether (1:1) to obtain 2.2 g. of the product.

It was confirmed that the product was 1-(4-bromodifluoromethylsulfonylphenylcarbamoyl)-3-(4-difluoromethoxyphenyl)-4-phenyl-2-pyrazoline (melting point of 159—162°C) by the NMR spectrum (XI).

Preparation 12:

1-(4-Bromodifluoromethoxyphenylcarbamoyl)-3-(4-difluoromethoxyphenyl)-4-(4-fluorophenyl)-2-pyrazoline

*Preparation of Compound No. 104*

(a) *Preparation of 3-(4-difluoromethoxyphenyl)-4-(4-fluorophenyl)-2-pyrazoline*

In accordance with the process (a) of Preparation 1 except using 18.4 g. of 4-fluorobenzyl-4'-(hydroxy) phenyl ketone instead of benzyl-p-(hydroxy)phenyl ketone, 4-fluorobenzyl-4'-(difluoromethoxy) phenyl ketone (melting point of 50—56°C) as an intermediate was obtained. Then, 4-difluoromethoxy-2-(4-fluorophenyl)acrylophenone ($N_D^{20}$ 1.5594) was produced by reacting formaline with the product.

Then, 15.2 g. of 3-(4-difluoromethoxyphenyl)-4-(4-fluorophenyl)-2-pyrazoline was produced by reacting hydrazine hydrate with the product. The reaction mixture was used as a reagent in the following step (c) without any purification.

(b) *Preparation of p-bromodifluoromethoxyphenylisocyanate*

Into DMF (20 ml.), 50% NaH (5.0 g. 104 m mol) was added and the mixture was cooled on an ice bath. A solution of p-nitrophenol (13.9 g. 100 m mol) in DMF (100 ml.) was added dropwise and the mixture was stirred for 10 minutes. A solution of dibromodifluoromethane (25 g. 119 m mol) in DMF (60 ml.) was added and the mixture was stirred for 5 hours at room temperature. The reaction mixture was poured into water and the product was extracted with ether. The ether layer was dried over anhydrous magnesium sulfate and the solvent was distilled off and the product was distilled to obtained 13.4 g. of p-nitro(bromodifluoromethoxy)benzene (boiling point of 74—77°C (0,133 mbar 0.1 mmHg)). The yield was 50%.

Into ethanol (100 ml.), the resulting p-(bromodifluoromethoxy)nitrobenzene (10.0 g.), iron powder (7.5 g.) and acetic acid (16 g.) were added. The mixture was refluxed for 2 hours. After distilling off most of ethanol, water was added and the product was extracted with ether. The ether layer was washed with NaCl aq. sol. and dried over anhydrous magnesium sulfate. The solvent was removed and the product was distilled to obtain 6.2 g. of p-(bromodifluoromethoxy)aniline (boiling point of 65—72°C 0.133 mbar (0.1 mmHg)). The yield was 70%.

Into 40 ml. of ethyl acetate, 4 g. of p-(bromodifluoromethoxy) aniline was dissolved. The solution was added dropwise to 40 ml. of ethyl acetate while feeding phosgen. After the addition, phosgen was further

fed for 10 min. The reaction mixture was concentrated under a reduced pressure to obtain 4 g. of the product ($N_D^{20}$ 1.4903).

The product was used as a reagent in the next step without any purification.

(c) *Preparation of Compound No. 104*

Into 20 ml. of anhydrous ethyl ether, 6.1 g. of 3-(4-difluoromethoxyphenyl)-4-(4-fluorophenyl)-2-pyrazoline obtained in the step (a) and 5.3 g. of p-bromodifluoromethoxyphenylisocyanate obtained in the step (b) were charged to react them as the step (b) of Preparation 10 to obtain 7.1 g. of 1-(4-bromodifluoromethoxyphenylcarbamoyl)-3-(4-difluoromethoxyphenyl)-4-(4-fluorophenyl)-2-pyrazoline (melting point of 121.5—123.5°C).

The formula of the product was confirmed by the NMR spectrum (XII).

Preparation 13:

1-(4-trifluoromethoxyphenylcarbamoyl)-3-(4-difluoromethoxyphenyl)-4-(4-chlorophenyl)-2-pyrazoline

*Preparation of Compound No. 90*

Into 20 ml. of anhydrous ethyl ether, 6.5 g. of 3-(4-difluoromethoxyphenyl)-4-(4-chlorophenyl)-2-pyrazoline and 4.0 g. of p-trifluoromethoxyphenylisocyanate were charged. The mixture was kept for one night and the precipitated crystal was separated by a filtration. The yield was 6.3 g.

It was confirmed that the product is 1-(4-trifluoromethoxyphenylcarbamoyl)-3-(4-difluoromethoxyphenyl)-4-(4-chlorophenyl)-2-pyrazoline (melting point of 117—119°C) by the NMR spectrum (XIII).

Preparation 14:

1-(4-Trifluoromethylthiophenylcarbamoyl)-3-(4-difluoromethoxyphenyl)-4-phenyl-5,5-dimethyl-2-pyrazoline

*Preparation of Compound No. 174*

(a) *Preparation of 3-(4-difluoromethoxyphenyl)-4-phenyl-5,5-dimethyl-2-pyrazoline*

Into a solution of 43 g. of benzyl-p-(difluoromethoxy)phenyl ketone obtained in the process (a) of Preparation 1 in 200 ml. of anhydrous tetrahydrofuran was cooled on an ice bath and 10 g. of NaH (55% in mineral oil) was added, and the mixture was stirred for 10 min. and then. 30 g of isopropyl iodide was added dropwise. After the addition, the mixture was refluxed for 4 hours.

After the reaction, tetrahydrofuran distilled off and 200 ml. of water and 200 ml. of ether were added to separate the organic phase by extraction. The water phase was treated with 100 ml. of ether by the extraction. The organic phases were mixed and dried over anhydrous sodium sulfate and the solvent was distilled off and the product was purified by a column chromatography (silica gel: benzene) to obtain 44 g. of 4′-difluoromethoxy-2-phenylisovalerophenone ($N_D^{20}$ = 1.5312). Into 20 ml. of carbon tetrachloride, 2.9 g. of the resulting product was dissolved and then, 0.54 ml. of bromine was added dropwise at room temperature and the mixture was stirred at room temperature for 24 hours. The reaction mixture was concentrated under a reduced pressure and 20 ml. of dimethylformamide was added to form a solution and 1.73 g. of LiCl was added and the mixture was heated at 130°C for 3 hours. Into the reaction mixture, 50 ml. of water and 50 ml. of ethyl ether were added and the organic phase was separated by a phase separation and was dried over anhydrous sodium sulfate, and ethyl ether was distilled off to obtain 2.7 g. of 4′-difluoromethoxy-α-phenyl-β,β-dimethyl acrylophenone ($N_D^{20}$ = 1.5623).

Into 20 ml. of methanol, 1.0 g. of the product and 1.7 ml. of hydrazine were dissolved. The reactor was wrapped with aluminum foil to be dark. The mixture was stirred at room temperature in nitrogen atmosphere for 24 hours. The reaction mixture was concentrated under a reduced pressure and 20 ml. of ethyl ether and 20 ml. of water were added. The organic phase was dried over anhydrous sodium sulfate and ethyl ether was distilled off to obtain 1.0 g. of the crude product. The product was used in the next step without any purification.

(b) *Preparation of Compound No. 174*

Into 20 ml. of anhydrous ethyl ether, 1.0 g. of the crude product obtained in the step (b) and 0.77 g. of p-trifluoromethylthiophenylisocyanate were charged to react them at room temperature for 24 hours. The precipitated by-product of N,N′-bis(4-trifluoromethylthiophenyl)urea was separated by a filtration and ethyl ether was distilled off under a reduced pressure to obtain 1.5 g. of the crude product. The crude product was admixed with 20 ml. of n-hexane-ethyl ether (1:1) and the precipitated crystal was separated by a filtration. The yield was 0.7 g.

It was confirmed that the product is 1-(4-trifluoromethylthiophenylcarbamoyl)-3-(4-difluoromethoxyphenyl)-4-phenyl-5,5-dimethyl-2-pyrazoline (melting point of 139.5—141°C) by the NMR spectrum (XIV).

Preparation 15:

1-(4-1′,1′,2′,2′-Tetrafluoroethoxyphenylcarbamonyl)-3-(4-difluoromethoxyphenyl)-2-pyrazoline

*Preparation of Compound No. 121*

Into 30 ml. of anhydrous ethyl ether, 4.2 g. of 3-(4-difluoromethoxyphenyl)-2-pyrazoline and 9.4 g. of p-1′,1′,2′,2′-tetrafluoroethoxyphenylisocyanate were charged and the mixture was kept at room temperature for one night and the precipitated crystal was separated by a filtration. The yield was 9.5 g.

It was confirmed that the product was 1-(4-1′,1′,2′,2′-tetrafluoroethoxyphenylcarbamoyl)-3-(4-difluoromethoxyphenyl)-2-pyrazoline (melting point of 101—103°C) by the NMR spectrum (XV).

Preparation 16:

1-(4-Chlorodifluoromethylthiophenylcarbamonyl)-3-(4-difluoromethoxyphenyl)-4-methyl-2-pyrazoline

*Preparation of Compound No. 126*

Into 20 ml. of anhydrous ethyl ether, 4.7 g. of 3-(4-difluoromethoxyphenyl)-4-methyl-2-pyrazoline and 4.7 g. of p-chlorodifluoromethylthiophenylisocyanate were charged and the mixture was kept at room temperature for one night, and ethyl ether was distilled off to obtain a crude product.

The crude product was purified by a silica gel thin layer chromatography (benzene: ethyl acetate = 4:1 as developer) to obtain 5.6 g. of a crystalline product.

It was confirmed that the product is 1-(4-chlorodifluoromethylthiophenylcarbamonyl)-3-(4-difluoromethoxyphenyl)-4-methyl-2-pyrazoline (melting point of 143—146°C) by the NMR spectrum (XVI).

Preparation 17:

1-(4-Trifluoromethoxyphenylcarbamonyl)-3-(4-difluoromethoxyphenyl)-4-isopropyl-2-pyrazoline

*Preparation of Compound No. 132*

(a) *Preparation of 3-(4-difluoromethoxyphenyl)-4-isopropyl-2-pyrazoline*

In accordance with the process of Preparation 5 except using 71.2 g. of p-hydroxyisovalerophenone instead of p-hydroxyacetophenone, p-difluoromethoxyisovalerophenone (boiling point of 103—110°C 0.665 mbar (0.5 mmHg)) as an intermediate was obtained. The product was dimethylaminomethylated to obtain 4'-difluoromethoxy-3-dimethylamino-2-isopropiophenone hydrochloride. The product was reacted with hydrazine hydrate in the presence of 50% NaOH aq. sol. to obtain 43 g. of 3-(4-difluoromethoxyphenyl)-4-isopropyl-2-pyrazoline. The product was used in the next step without any purification.

(b) *Preparation of Compound No. 132*

Into 30 ml. of anhydrous ethyl ether, 5.1 g. of 3-(4-difluoromethoxyphenyl)-4-isopropyl-2-pyrazoline and 4.0 g. of p-trifluoromethoxyphenylisocyanate were charged and the mixture was kept at room temperature for one night and ethyl ether was distilled off under a reduced pressure to obtain a crude product.

The crude product was purified by a silica gel thin layer chromatography (benzene: ethyl acetate = 4:1 as developer) to obtain 4.5 g. of a crystalline product.

It was confirmed that the product is 1-(4-trifluoromethoxyphenylcarbamoyl)-3-(4-difluoromethoxyphenyl)-4-isopropyl-2-pyrazoline (melting point of 103—107°C) by the NMR spectrum (XVII).

Preparation 18:

1-(4-Trifluoromethylthiophenylcarbamoyl)-3-(4-difluoromethoxyphenyl)-4-(3-cyanopropyl)-2-pyrazoline

*Preparation of Compound No. 148*

(a) *Preparation of 3-(4-difluoromethoxyphenyl)-4-(3-cyanopropyl)-2-pyrazoline*

Into a solution of 17 g. of NaOH in 160 ml. of water and 400 ml. of dioxane, 79.4 g. of 4'-hydroxy-5-chlorovalerophenone was added in Reactor A.

On the other hand, a solution of 80 g. of NaOH in 300 ml. of water and 360 ml. of dioxane was prepared in Reactor B. The Reactor B was heated at 80°C, Freon®-22 gas was fed and the resulting difluorocarbene was fed through a polytetrafluoroethylene tube (previously connected) into the Reactor A. In the Reactor A, the temperature was raised. After feeding 200 g. of Freon®-22, the Reactor A was cooled and 400 ml. of water and 500 ml. of ethyl ether were charged to separate an organic phase by extraction. The organic phase was dried over anhydrous sodium sulfate and ethyl ether was distilled off as a crude product of 4'-difluoromethoxy-5-chlorovalerophenone.

Into 20 ml. of acetophenone, 13 g. of the crude product was dissolved and 6.5 g. of potassium cyanide and 1 g. of 18-crown-6 were added to the solution and the mixture was refluxed for 3 hours. After cooling the reaction mixture, an insoluble matter was separated by a filtration and 50 ml. of water was added to the filtrate. The product was extracted two times with 50 ml. of chloroform and the chloroform phase was dried over anhydrous sodium sulfate and the solvent was distilled off under a reduced pressure to obtain 12 g. of the crude product. The crude product was purified by a column chromatography (silica gel: ethyl acetate: benzene = 1:9 as developer) to obtain 9 g of 4'-difluoromethoxy 5-cyanovalerophenone ($N_D^{20}$ 1.5045). The product (4.8 g.) was admixed with 2 g. of dimethylamine hydrochloride, 0.8 g. of paraformaldehyde and 20 ml. of dioxane and the mixture was refluxed for 24 hours. After distilling off dioxane, 30 ml. of water was added and the product was extracted two times with 30 ml. of ether. The ether phase was dried over anhydrous sodium sulfate and ether was distilled off to obtain 4.7 g. of 4'-difluoromethoxy-α-(3-cyanopropyl)acrylophenone.

The product was dissolved in 50 ml. of methanol and then 3 ml. of hydrazine hydrate was added and the mixture was refluxed for 3 hours. After distilling off methanol, 30 ml. of water was added and the product was extracted two times with 30 ml. of ether and the ether phase was dried over anhydrous sodium sulfate and ether was distilled off to obtain 4.8 g. of 3-(4-difluoromethoxyphenyl)-4-(3-cyanopropyl)-2-pyrazoline. The product was used in the next step without any purification.

(b) *Preparation of Compound No. 148*

Into 20 ml. of anhydrous ethyl ether, 5.6 g. of 3-(4-difluoromethoxyphenyl)-4-(3-cyanopropyl)-2-pyrazoline obtained in the step (a) and 4.4 g. of p-trifluoromethylthiophenylisocyanate were charged and the mixture was kept at room temperature for one night and ethyl ether was distilled off under a reduced pressure to obtain a crude product. The product was purified by a silica gel thin chromatography (benzene-ethyl acetate = 4:1) to obtain 4 g. of a crystalline product.

It was confirmed that the product is 1-(4-trifluoromethylthiophenylcarbamoyl)-3-(4-difluoromethoxyphenyl)-4-(3-cyanopropyl)-2-pyrazoline (melting point of 137.5—139°C) by the NMR spectrum (XVIII).

Preparation 19:

1-(4-Trifluoromethoxyphenylcarbamoyl)-3-(4-trifluoromethoxyphenyl)-4-phenyl-2-pyrazoline

*Preparation of Compound No. 156*

(a) *Preparation of benzyl-p-(trifluoromethoxy)phenyl ketone*

Into 50 ml. of dimethylformamide, 9.0 g. of 50% NaH was added and the mixture was cooled on an ice bath and a solution of 21 g. of p-cyanophenol in 100 ml. of dimethylformamide was added dropwise and the mixture was stirred for 10 min. and then, a solution of 60 g. of dibromodifluoromethane in 80 ml. of dimethylformamide was added and the mixture was stirred at room temperature for one day. The reaction mixture was poured into water and the product was extracted with ether. The ether phase was dried over anhydrous magnesium sulfate and the solvent was distilled off and the product was distilled to obtain 13.8 g. of p-(bromodifluoromethoxy)benzonitrile (boiling point of 64—66°C 0.133 mbar (0.1 mmHg)). The yield was 33%. Into 40 ml. of isopropyl ether, 13.4 g. of p-(bromodifluoromethoxy) benzonitrile was dissolved and 20 ml. of a solution of HF pyridine (70:30) was added and then 100 mg. of mercury oxide was added after 1 hour and the mixture was continuously stirred. After confirming the end point of the reaction by a gas chromatography, the reaction mixture was poured into water and the product was extracted with ether. The ether phase was washed with NaOH aq. sol. and then with NaCl aq. sol. and dried over anhydrous magnesium sulfate and the solvent was distilled off to obtain 7.6 g. of p-(trifluoromethoxy)-benzonitrile (boiling point of 89—92°C/22.6 mbar (17 mmHg.)). A Grignard reagent was prepared by using 10 g. of benzyl chloride and 1.9 g. of magnesium in 60 ml. of ether. A solution of 7.5 g. of p-(trifluoro-methoxy)benzonitrile in 30 ml. of ether was added to the Grignard reagent and the mixture was refluxed for 3 hours. The reaction mixture was poured into a diluted hydrogen chloride and the product was extracted with ether and ether was removed and the product was recrystallized from hexane to obtain 9.3 g. of benzyl-p-(trifluoromethoxy)phenyl ketone (melting point of 86—87°C) ($^1H$—NMR: δ 4.27 (2H, S), 7.32 (7H), 8.08 (2H, d), $^{19}F$—NMR: 21.2 (S)).

(b) *Preparation of 3-(4-trifluoromethoxyphenyl)-4-phenyl-2-pyrazoline*

Into a mixture of 0.8 ml. of piperidine, 0.8 ml. of acetic acid, 23 ml. of 37% formaline and 160 ml. of methanol, 16.8 g. of benzyl-p-(trifluoromethoxy)phenyl ketone obtained in the step (a) was added and the mixture was refluxed for 3 hours. The reaction mixture was concentrated under a reduced pressure and was admixed with 130 ml. of water and 200 ml. of chloroform and the organic phase was obtained by a phase separation. The organic phase was dried over anhydrous sodium sulfate and chloroform was distilled off to obtain 17.0 g. of 4′-(trifluoromethoxy)-2-phenylacrylophenone.

A mixture of 14.6 g. of the resulting product, 7 ml. of hydrazine hydrate and 130 ml. of ethanol was refluxed for 3 hours. After the reaction, the reaction mixture was concentrated under a reduced pressure and then, 70 ml. of water and 100 ml. of chloroform were added to obtain an organic phase. The organic phase was dried over anhydrous sodium sulfate and chloroform was distilled off to obtain 14.9 g. of 3-(4-trifluoromethoxyphenyl)-4-phenyl-2-pyrazoline (melting point of 90—102°C). The product was used in the next step without any purification.

(c) *Preparation of Compound No. 156*

Into 100 ml. of anhydrous ethyl ether, 3.1 g of 3-(4-trifluoromethoxyphenyl)-4-phenyl-2-pyrazoline obtained in the step (b) and 2.0 g. of p-trifluoromethoxyphenylisocyanate were charged to react them at room temperature for 24 hours. The precipitated crystal was separated by a filtration. The yield was 3.8 g.

It was confirmed that the product is 1-(4-trifluoromethoxyphenylcarbamoyl)-3-(4-trifluoromethoxyphenyl)-4-phenyl-2-pyrazoline (melting point of 141.0—143.5°C) by the NMR spectrum (XIX).

Preparation 20:

1-(4-Chlorophenylcarbamoyl)-3-(4-bromodifluoromethoxyphenyl)-4-phenyl-2-pyrazoline

*Preparation of Compound No. 160*

(a) *Preparation of benzyl-p-(bromodifluoromethoxy)phenyl ketone*

In accordance with the process (a) of Preparation 19, the product was produced as follows:

A solution of 13.8 g. of p-(bromodifluoromethoxy)benzonitrile in 30 ml. of ether was added to a Grignard reagent obtained by using 60 ml. of ether, 10 g. of benzyl chloride and 1.9 g. of magnesium. The mixture was refluxed for 3 hours. After the reaction, the product was purified in accordance with the process (a) of Preparation 19 to obtain 12.3 g. of benzyl-p-(bromodifluoromethoxy)phenyl ketone.

(b) *Preparation of 3-(4-bromodifluoromethoxyphenyl)-4-phenyl-2-pyrazoline*

In accordance with the process of Preparation 19 except using 20.5 g. of benzyl-p-(bromodifluoromethoxy)phenyl ketone obtained in the step (a) instead of 16.8 g. of benzyl-p-(trifluoromethoxy)phenyl ketone, a production was carried out to obtain 20.8 g. of 4'-(bromodifluoromethoxy-2-phenylacrylophenone ($N_D^{20}$ 1.5723).

A mixture of 17.7 g. of the resulting product, 7 ml. of hydrazine hydrate and 130 ml. of ethanol was refluxed for 3 hours. After the reaction, the reaction mixture was concentrated under a reduced pressure and 70 ml. of water and 100 ml. of chloroform were added to obtain an organic phase. The organic phase was dried over anhydrous sodium sulfate and chloroform was distilled to obtain 17.9 g. of 3-(4-bromodifluoromethoxyphenyl)-4-phenyl-2-pyrazoline. The product was used in the next step without any purification.

(b) *Preparation of Compound No. 160*

Into 50 ml. of anhydrous ethyl ether, 3.7 g. of 3-(4-bromodifluoromethoxyphenyl)-4-phenyl-2-pyrazoline obtained in the step (b) and 1.5 g. of p-chlorophenylisocyanate were charged to react them at room temperature for 24 hours. The precipitated crystal was separated by a filtration. The yield was 3.9 g.

It was confirmed that the product is 1-(4-chlorophenylcarbamoyl)-3-(4-bromodifluoromethoxyphenyl)-4-phenyl-2-pyrazoline (melting point of 132.0—134.5°C) by the NMR spectrum (XX).

Preparation 21:

1-N-(4-chlorophenyl)-N-methyl-carbamoyl-3-(4-difluoromethoxyphenyl)-4-phenyl-2-pyrazoline

*Preparation Compound No. 167*

Into 12.5 ml. of DMF, 2.2 g. of Compound No. 7 obtained by the process of Preparation 1 was dissolved on an ice bath and a solution of 0.35 g. of KOH in 0.5 ml. of water was added to the solution. After stirring the mixture for 10 min., 0.71 g. of methyl iodide was added to react them at room temperature for 3 hours.

After the reaction, the solution was poured into water and the product was extracted two times with 50 ml. of chloroform. The organic phase was dried over anhydrous sodium sulfate and the solvent was distilled off to obtain 2.3 g. of a crude product. The crude product was purified by a column chromatography and the product was recrystallized from a mixture of ether and n-hexane (1:1) to obtain 1.0 g. of 1-N-(4-chlorophenyl)-N-methylcarbamoyl-3-(4-difluoromethoxyphenyl)-4-phenyl-2-pyrazoline (melting point of 103—107°C) which was confirmed by the NMR spectrum (XXI).

NMR spectra (I)—(XXI)

$^{1}H$—NMR: δ($CDCl_3$. TMS, ppm)

| NMR spectrum | |
|---|---|
| I | 3.85—4.90 (3H, m); 6.46 (1H, t, J=73Hz); 7.23 (5H, s); 6.90—7.80 (8H, m); 8.20 (1H, bs) |
| II | 3.90—4.95 (3H, m); 6.55 (1H, t, J=73Hz); 7.34 (5H, s); 7.00—8.00 (7H, m); 8.30 (1H, bs) |
| III | 4.25—4.80 (3H, m); 6.45 (1H, t, J=73Hz); 7.20 (5H, s); 6.90—7.80 (8H, m); 9.10 (1H, bs) |
| IV | 3.85—4.90 (3H, m); 6.48 (1H, t, J=73Hz); 6.95—7.80 (12H, m); 8.27 (1H, bs) |
| V | 3.00—3.40 (2H, m); 3.75 (3H, s); 3.85—4.30 (2H, m); 6.55 (1H, t, J=73Hz); 6.75—7.90 (9H, m) |
| VI | 1.25 3H, d, J=6.09Hz); 3.45—4.30 (3H, m); 6.56 (1H, t, J=73Hz); 7.00—7.90 (8H, m); 8.05 (1H, bs) |
| VII | 2.90—4.10 (2H, m); 5.45—5.70 (1H, m); 6.56 (1H, t, J=73Hz); 7.27 (4H, s); 7.38 (4H, s); 7.05—7.85 (4H, m); 8.07 (1H, bs) |
| VIII | 3.90—4.90 (3H, m); 6.50 (1H, t, J=73Hz); 7.30 (5H, s); 7.67 (4H, s); 7.00—7.80 (4H, m); 8.30 (1H, bs) |
| IX | 3.90—4.95 (3H, m); 6.49 (1H, t, J=73Hz); 7.29 (5H, s); 7.80 (4H, s); 6.90—7.85 (4H, m); 8.40 (1H, bs) |
| X | 3.90—4.90 (3H, m); 6.50 (1H, t, J=73Hz); 7.30 (5H, s); 7.66 (4H, s); 6.95—7.85 (4H, m); 8.30 (1H, bs) |
| XI | 3.90—4.85 (3H, m); 6.53 (1H, t, J=73Hz); 7.33 (5H, s); 7.00—7.85 (4H, m); 7.98 (4H, s); 8.60 (1H, bs) |
| XII | 3.80—4.90 (3H, m); 6.53 (1H, t, J=73Hz); 6.90—7.80 (12H, m); 8.20 (1H, bs) |
| XIII | 3.80—4.90 (3H, m); 6.49 (1H, t, J=73Hz); 6.90—7.80 (12H, m); 8.17 (1H, bs) |
| XIV | 1.28 (3H, s); 1.63 (3H, s); 4.25 (1H, s); 6.48 (1H, t, J=73Hz); 6.95—7.75 (13H, m); 8.43 (1H, bs) |
| XV | 2.95—3.45 (2H, m); 3.85—4.30 (2H, m); 5.90 (1H, tt, J=54.0Hz and 3.0Hz); 6.57 (1H, t, J=73Hz); 7.00—7.90 (8H, m); 8.07 (1H, bs) |
| XVI | 1.30 (3H, d, J=6.0Hz); 3.45—4.25 (3H, m); 6.59 (1H, t, J=73Hz); 7.19 (2H, d, J=9.0Hz); 7.60 (4H, s); 7.78 (2H, d, J=9.0Hz); 8.20 (1H, bs) |
| XVII | 0.72 (3H, d, J=7.0Hz); 1.03 (3H, d, J=7.0Hz); 2.10 (1H, m); 3.70—4.20 (3H, m); 6.59 (1H, t, J=73Hz); 7.05—7.90 (8H, m) 8.10 (1H, bs) |
| XVIII | 1.50—1.90 (4H, m); 2.37 (2H, t, J=5.0Hz); 3.50—4.20 (3H, m); 6.59 (1H, t, J=73Hz); 7.61 (4H, s); 7.10—7.85 (4H, m); 8.17 (1H, bs) |
| XIX | 3.90—4.90 (3H, m); 7.00—7.85 (8H, m); 7.28 (5H, s); 8.15 (1H, bs) |
| XX | 3.85—4.90 (3H, m); 7.28 (5H, s); 7.00—7.85 (8H, m); 8.15 (1H, bs) |
| XXI | 3.39 (3H, s); 3.80—4.65 (3H, m); 6.40 (1H, t, J=73Hz); 6.75—7.50 (13H, m) |

Certain examples of the compositions of the compounds of the present invention as insecticides are provided for purposes of illustration only.

*Composition 1 Emulsifiable concentrate:*

| | |
|---|---|
| Active ingredient: | 10 wt.parts |
| Xylene: | 80 wt. parts |
| Sorpol 2680 (Toho Chem.): | 10 wt.parts |

The components were uniformly mixed to prepare an emulsifiable concentrate. The emulsifiable concentrate was diluted with water to 50—100,000 times and it was sprayed in amounts of 10—500 liter/10 ares.

As the active ingredient, Compound No. 1, 4, 6, 7, 9, 12, 16, 20, 25, 28, 38, 40, 44, 53, 54, 59, 61, 64, 65, 68, 69, 74, 77, 84, 85, 86, 87, 90, 92, 93, 94, 97, 99, 100, 103, 104, 106, 109, 110, 111, 112, 113, 114, 115, 116, 117, 121, 122, 123, 124, 125, 128, 132, 134, 135, 136, 137, 138, 139, 140, 141, 142, 143, 144, 148, 149 and 155 were used.

*Composition 2 Oil solution:*

| | |
|---|---|
| Active ingredient: | 50 wt.parts |
| Methyl cellosolve: | 50 wt.parts |

The components were uniformly mixed to obtain an oily solution.

The oil solution was applied in amounts of 0.1 to 50 ml./m$^2$ to a drain or puddle or in amounts of 10—100 ml./10 ares by airplain spray. As the active ingredient, Compound No. 7, 10, 54, 68, 74, 87, 92, 94, 103 and the other compounds in Tables were used.

*Composition 3 Wettable powder:*

| | |
|---|---|
| Active ingredient: | 25 wt.parts |
| Zeeklite PFP: | 65 wt.parts |
| Carplex #80: | 2 wt.parts |
| Sorpol 5050: | 2 wt.parts |
| Sodium ligninesulfonate: | 6 wt.parts |

The components were uniformly ground and mixed to obtain a wettable powder. The wettable powder was diluted with 100 to 250,000 times of water and it was sprayed in amounts of 20 to 500 liter/10 ares.

As the active ingredient, Composition No. 84, 92 and the other compounds in Tables were used.

*Composition 4 Dust:*

| | |
|---|---|
| Active ingredient: | 3.0 wt.parts |
| Carplex #80: | 0.5 wt.parts |
| Clay: | 95 wt.parts |
| Diisopropyl phosphate: | 1.5 wt.parts |

The components were uniformly mixed to obtain a dust. The dust was applied in amounts of 0.03 to 15 kg/10 ares.

As the active ingredient, Composition No. 9 and other compounds in Tables were used.

*Composition 5 Bait Poison:*

| | |
|---|---|
| Wheat bran: | 52 wt.parts |
| Rice bran: | 15 wt.parts |
| Wheat powder: | 30 wt.parts |
| Raw sugar (muscovado): | 3 wt.parts |

The components were uniformly mixed and each active ingredient was added at a ratio of 0.2% base on the total components. Water was added at a ratio of 50% based on the total components and the mixture was granulated by a pelleter and dried at 50 to 60°C by hot air. The resulting bait poison was placed in amounts of 0.1—5 g./m$^2$ around a root of a plant.

As the active ingredient, Compound No. 1, 4, 7, 9, 10, 16, 21, 25, 28, 38, 40, 44, 53, 54, 56, 59, 61, 63—66, 68, 69, 71—75, 77, 81, 84—88, 90, 92—99, 100, 102, 103, 106, 110—117, 120, 122, 123, 124, 126, 127, 130, 133, 134—144, 148 and 159 were used.

The insecticidal activities of the compounds of the present invention will be illustrated by tests.

Experiment 1

*Contact test for killing adult houseflies:*

A 1 ml. of 100 ppm solution of each active ingredient of the present invention in acetone was dropped onto the bottom of a Petri dish (9 cm) and was spread uniformly over the surface of the dish. Acetone was completely evaporated at room temperature. Ten adult houseflies were placed in the dish, which was covered with a plastic cover having many holes. The Petri dish was maintained in a constant temperature room at 25°C for 48 hours and each percent mortality of the houseflies was determined. The test was repeated twice and the results are shown in Table 5.

TABLE 5

| Active ingredient | Percent mortality (%) |
|---|---|
| Compound No. 7 | 100 |
| No. 9 | 100 |
| No. 12 | 95 |
| No. 16 | 100 |
| No. 44 | 100 |
| No. 53 | 100 |
| No. 54 | 100 |
| No. 66 | 100 |
| No. 74 | 100 |
| No. 84 | 100 |
| No. 85 | 100 |
| No. 86 | 100 |
| No. 90 | 100 |
| No. 92 | 100 |
| No. 93 | 100 |
| No. 94 | 100 |

TABLE 5 (continued)

| Active ingredient | Percent mortality (%) |
|---|---|
| No. 96 | 100 |
| No. 103 | 100 |
| No. 106 | 100 |
| No. 122 | 100 |
| No. 123 | 100 |
| No. 152 | 100 |
| No. 153 | 100 |
| No. 154 | 100 |
| No. 155 | 100 |
| No. 156 | 100 |
| No. 157 | 100 |
| No. 158 | 100 |
| No. 159 | 100 |
| No. 160 | 100 |
| No. 162 | 100 |
| No. 163 | 100 |
| No. 166 | 100 |

Experiment 2

*Contact test for killing green rice leafhopper*

Stems and leaves of a rice seedling were dipped in each emulsion of each composition of the active ingredient of the present invention (100 ppm) for 10 seconds and were dried in air. The stems and leaves were covered with a glass cylinder adult green rice leaf-hoppers which are resistant to the conventional organic phosphorus type insect pesticides were released into the glass cylinder which was covered with a cover having holes and was maintained in a constant temperature toom at 25°C for 48 hours and each percent mortality was determined.-

The results are shown in Table 6.

TABLE 6

| Active ingredient | Percent mortality (%) |
|---|---|
| Compound No. 7 | 100 |
| No. 9 | 100 |
| No. 16 | 100 |
| No. 44 | 100 |
| No. 54 | 100 |
| No. 75 | 100 |
| No. 77 | 100 |
| No. 153 | 100 |
| No. 155 | 100 |
| No. 159 | 100 |
| No. 161 | 100 |
| No. 167 | 100 |
| Reference Comp. A | 20 |

Note:
Reference Compound A:

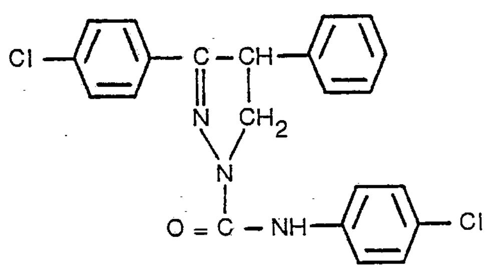

(Japanese Unexamined Patent Publication No. 41358/1976)

Experiment 3
*Contact test for killing Common cutworm*

Leaves of cabbage were dipped in each aqueous emulsion of each active ingredient of the compounds of the invention or the reference for 10 seconds. The leaves were taken up and dried in air and put in a Petri dish. Common cutworms (second instar) were put in the Petri dish which was covered with a cover having many holes. The Petri dish was maintained in a constant temperature room at 25°C for 48 hours and each percent mortality was determined. The results are shown in Table 7.

TABLE 7—1

| Active ingredient | Concentration (ppm) | Percent mortality (%) |
|---|---|---|
| Compound No. 7 | 1.25 | 100 |
| No. 9 | 1.25 | 100 |
| No. 10 | 1.25 | 100 |
| No. 16 | 1.25 | 100 |
| No. 21 | 1.25 | 100 |
| No. 41 | 1.25 | 100 |
| No. 54 | 1.25 | 100 |
| No. 59 | 1.25 | 100 |
| No. 64 | 1.25 | 100 |
| No. 65 | 1.25 | 100 |
| No. 66 | 1.25 | 100 |
| No. 68 | 1.25 | 100 |
| No. 71 | 1.25 | 100 |
| No. 72 | 1.25 | 100 |
| No. 73 | 1.25 | 100 |
| No. 74 | 1.25 | 100 |
| No. 75 | 1.25 | 100 |
| No. 77 | 1.25 | 100 |
| No. 81 | 1.25 | 100 |
| No. 82 | 1.25 | 100 |
| No. 84 | 1.25 | 100 |
| No. 85 | 1.25 | 100 |
| No. 86 | 1.25 | 100 |
| No. 88 | 1.25 | 100 |
| No. 90 | 1.25 | 100 |
| No. 92 | 1.25 | 100 |
| No. 93 | 1.25 | 100 |
| No. 94 | 1.25 | 100 |
| No. 96 | 1.25 | 100 |

TABLE 7—1 (continued)

| Active ingredient | Concentration (ppm) | Percent mortality (%) |
|---|---|---|
| Compound No. 98 | 1.25 | 100 |
| No. 99 | 1.25 | 100 |
| No. 100 | 1.25 | 100 |
| No. 102 | 1.25 | 100 |
| No. 103 | 1.25 | 100 |
| No. 104 | 1.25 | 100 |
| No. 106 | 1.25 | 100 |
| No. 152 | 1.25 | 100 |
| No. 153 | 1.25 | 100 |
| No. 155 | 1.25 | 100 |
| No. 156 | 1.25 | 100 |
| No. 157 | 1.25 | 100 |
| No. 158 | 1.25 | 100 |
| No. 159 | 1.25 | 100 |
| No. 160 | 1.25 | 100 |
| No. 162 | 1.25 | 100 |
| No. 163 | 1.25 | 100 |
| Reference Comp. A | 1.25 | 70 |

TABLE 7—2

| Active ingredient | Concentration (ppm) | Percent mortality (%) |
|---|---|---|
| Compound No. 110 | 10 | 100 |
| No. 121 | 10 | 100 |
| No. 122 | 10 | 100 |
| No. 123 | 10 | 100 |
| No. 124 | 10 | 100 |
| No. 126 | 10 | 100 |
| No. 127 | 10 | 100 |
| No. 130 | 10 | 100 |
| No. 134 | 10 | 100 |
| No. 137 | 10 | 100 |
| No. 142 | 10 | 100 |
| No. 143 | 10 | 100 |
| No. 144 | 10 | 100 |
| No. 148 | 10 | 100 |
| No. 149 | 10 | 100 |
| Reference Comp. B | 10 | 40 |
| C | 10 | 70 |
| D | 10 | 60 |

Note:
Reference Compound A:

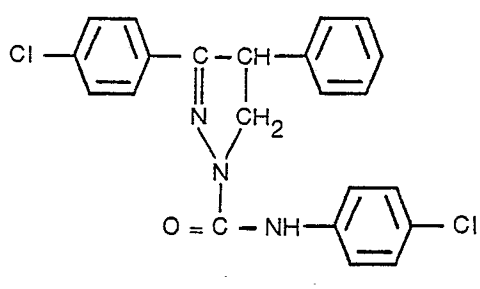

(Japanese Unexamined Patent Publication No. 41358/1976)

Reference Compound B:

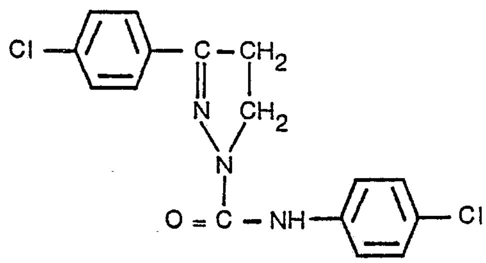

(Japanese Unexamined Patent Publication No. 87028/1973)

Reference Compound C:

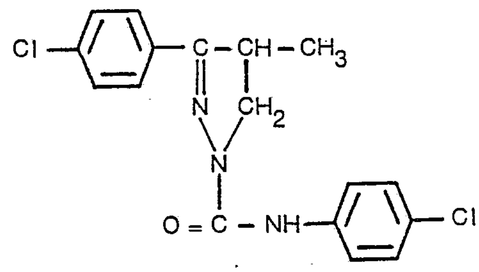

(Japanese Unexamined Patent Publication No. 41358/1976)

Reference Compound D:

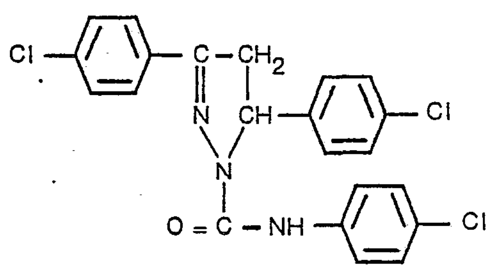

(Japanese Unexamined Patent Publication No. 87028/1973)

Experiment 4

*Contact test for killing Twenty-eight-spotted ladybeetle*

Leaves of tomato were dipped in 1 ppm aqueous emulsion of each active ingredient of the compounds of the present invention and the reference for 10 seconds. The leaves were taken up and dried in air and put in a Petri dish. Ten of Twenty-eight-spotted Ladybeetles (second instar) were put in the Petri dish which was covered with a cover. The Petri dish was maintained in a constant temperature room at 25°C for 48 hours and percent mortality was determined. The tests were carried out in two groups. The results are shown in Table 8.

TABLE 8

| Active ingredient | Percent mortality (%) |
|---|---|
| Compound No. 7 | 100 |
| No. 9 | 100 |
| No. 16 | 100 |
| No. 44 | 100 |
| No. 54 | 100 |
| No. 59 | 100 |
| No. 62 | 100 |
| No. 64 | 100 |
| No. 65 | 100 |
| No. 68 | 100 |
| No. 71 | 100 |
| No. 74 | 100 |
| No. 75 | 100 |
| No. 77 | 100 |
| No. 78 | 100 |
| No. 81 | 100 |
| No. 82 | 100 |
| No. 84 | 100 |
| No. 85 | 100 |
| No. 86 | 100 |
| No. 90 | 100 |
| No. 92 | 100 |
| No. 93 | 100 |
| No. 94 | 100 |
| No. 99 | 100 |
| No. 100 | 100 |
| No. 101 | 100 |
| No. 103 | 100 |
| No. 104 | 100 |

TABLE 8 (continued)

| Active ingredient | Percent mortality (%) |
|---|---|
| Compound No. 127 | 100 |
| No. 153 | 100 |
| No. 155 | 100 |
| No. 156 | 100 |
| No. 157 | 100 |
| No. 158 | 100 |
| No. 162 | 100 |
| No. 166 | 100 |
| Reference Comp. A | 70 |

Note:
Reference Compound A:

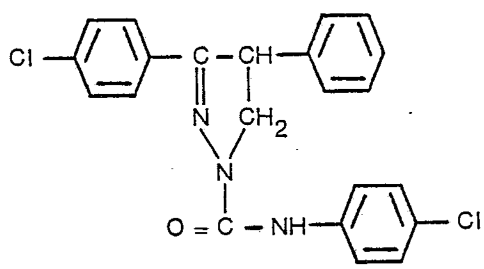

(Japanese Unexamined Patent Publication No. 41358/1976)

Experiment 5
*Test for killing diamond back moth*

Leaves of cabbage were dipped in each aqueous emulsion of each active ingredient of the compounds of the invention or the reference (1 ppm) for 10 seconds. The leaves were taken up and dried in air and put in a Petri dish. Lavae of Diamond back moth were put in the Petri dish wich was covered with a cover having many holes. The Petri dish was maintained in a constant temperature room at 25°C for 48 hours and each percent mortality was determined. The results are shown in Table 9.

TABLE 9

| Active ingredient | Percent mortality (%) |
|---|---|
| Compound No. 7 | 100 |
| No. 54 | 100 |
| No. 64 | 100 |
| No. 68 | 100 |
| No. 69 | 100 |
| No. 71 | 100 |
| No. 72 | 100 |
| No. 74 | 100 |
| No. 75 | 100 |
| No. 77 | 100 |
| No. 81 | 100 |
| No. 82 | 100 |
| No. 87 | 100 |
| No. 90 | 100 |
| No. 92 | 100 |
| No. 94 | 100 |
| No. 100 | 100 |
| No. 101 | 100 |
| No. 102 | 100 |
| No. 103 | 100 |
| No. 104 | 100 |
| No. 153 | 100 |
| No. 155 | 100 |
| No. 156 | 100 |
| No. 157 | 100 |
| No. 158 | 100 |

TABLE 9 (continued)

| Active ingredient | Percent mortality (%) |
|---|---|
| Compound No. 159 | 100 |
| No. 160 | 100 |
| No. 162 | 100 |
| No. 164 | 100 |
| No. 165 | 100 |
| Reference Comp. A | 30 |

Note:
Reference Compound A:

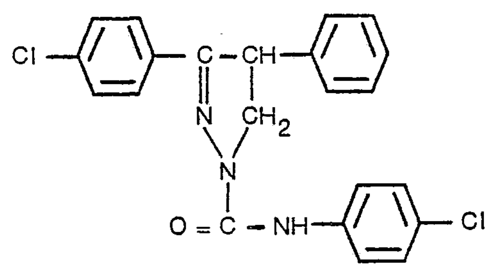

(Japanese Unexamined Patent Publication No. 41358/1976)

Experiment 6
*Bait poison test for killing Common cutworm*

Into each 1/5000 are pot, planting cabbage at 4 leaf stage, each bait poison composition 5 containing each active ingredient was put in an amount of 3 kg. or 6 kg./10 ares. Common cutworm (fifth instar) were put in the pot. The pot was maintained in a constant temperature room at 25°C. After 5 hours, the percent mortality for each of the active ingredients was 100%.

Experiment 7
*Contact test for killing Common cutworms (second instar): (mixed insect pesticide)*

Leaves of cabbage were dipped in aqueous emulsion of each single active ingredient or each mixture of Compound No. 7 and EPN or Fenvalerate (2:1) for 10 seconds. The leaves were taken up and dried in air and put in a Petri dish. Ten of Common cutworm (second instar) were put in a Petri dish which was covered with a cover having many holes. The Petri dish was maintained in a constant temperature room at 25°C for 72 hours and percent mortality was determined.

$LC_{50}$ (ppm) was determined by Finny equation. a synergistic coefficient was calculated from $LC_{50}$ (ppm) by the Sun method (J. Econ. Ent. *53* 5, 887—892 (1960)) to evaluate the synergistic effect for insect pesticides. The results are shown in Table 10.

TABLE 10

| | $LC_{50}$ (ppm) | | Synergistic coefficient |
|---|---|---|---|
| | Single Compound | Mixture of Compound 7 and other (2 : 1) | |
| Compound No. 7 | 0.29 | — | — |
| EPN | 1.1 | 0.22 | 192 |
| Fenvalerate | 1.7 | 0.22 | 182 |

The remarkable synergistic effects of the combination of Compound No. 7 and EPN or Fenvalerate were found.

Experiment 8

*Soil residual test*

An upland soil (50 g. as oven dry weight) was uniformly mixed with 1 ml. of each acetone solution of the Compound No. 7 or the Reference (50 ppm) in a 200 ml. beaker. The top of the beaker was covered with aluminum foil and the beaker was maintained in the dark at 30%. After the predetermined time, the compound was extracted with hydrated acetone from the soil and was purified and measured by high pressure liquid chromatography. The precent of residue of the compound in the soil was calculated. The results are shown in Table 11.

TABLE 11

| Compound | | Compound No. 7 | Reference A |
|---|---|---|---|
| Soil | | Tochigi volcanic ashes soil (Clay loam) | ,, |
| Water ratio of soil during the test (%) | | 60 | 60 |
| Fercent of residue of compound in soil (%) *1 | 30 day | 58 | 80 |
| | 90 day | 36 | 62 |

Note: *1

$$\text{Percent of residue} = \frac{\text{Content of compound in soil after predetermined day (ppm)}}{\text{Content of compound in soil at time of mixing (ppm)}} \times 100$$

The percent of residue of Compound No. 7 was smaller than that of Reference A in the test result. The residue in soil of Compound No. 7 was shorter than that of Reference A.

Note:

Reference Compound A:

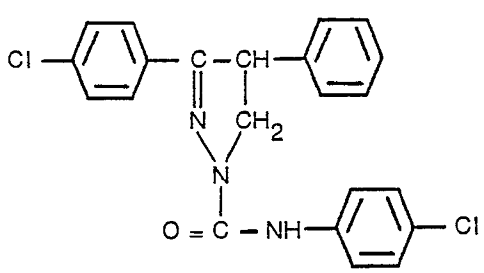

(Japanese Unexamined Patent Publication No. 41358/1976)

Experiment 9

*Fish-toxicity to kill fish:*

In a glass vessel having a diameter of 20 cm and a height of 10 cm, 2 liter of water was charged. A solution of each compound of the invention or the reference in methanol was added to give a concentration of 0.5 ppm. The vessel was maintained in a constant temperature tank at 25°C and ten of kill fish were put in the vessel and timely observed. Table 12 shows survival percents after 72 hours.

TABLE 12

| Active ingredient | Survival percent (%) |
|---|---|
| Compound No. 16 | 100 |
| No. 64 | 100 |
| No. 78 | 100 |
| No. 75 | 100 |
| No. 81 | 100 |
| No. 82 | 100 |
| No. 87 | 90 |
| No. 88 | 100 |
| No. 96 | 100 |
| No. 106 | 100 |
| Reference A | 0 |

Note:
Reference Compund A:

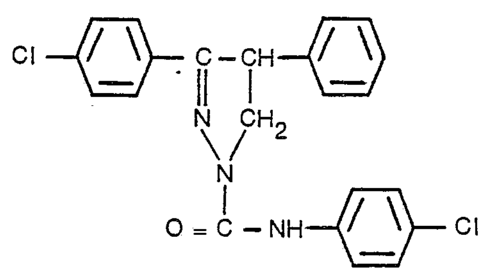

(Japanese Unexamined Patent Publicatoin No. 41358/1976)

**Claims**

1. Pyrazoline derivatives having the formula

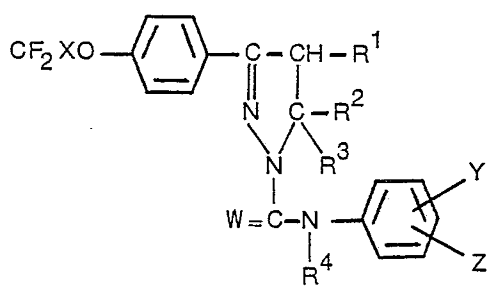

[I]

wherein $R^1$ represents hydrogen atom, a $C_{1-4}$-alkyl group, phenyl group, a halogen-substituted phenyl group, a cyano alkyl group or a $C_{1-4}$-alkoxy alkyl group; $R^2$ and $R^3$ respectively represent hydrogen atom, a $C_{1-4}$- alkyl group, phenyl group or a halogen-substituted phenyl group; $R^4$ represents hydrogen atoms or a

$C_{1-4}$-alkyl group; X represents hydrogen atom or a halogen atom; W represents oxygen or sulfur atom; Y and Z respectively represent hydrogen atom, a halogen atom, a $C_{1-4}$-alkyl group, a $C_{1-4}$-alkoxy group, nitro group, trifluoromethyl group, a $C_{1-4}$-alkylthio group, an acetyl group, nitrile group, a $C_{1-4}$-alkyl sulfonyl group, a $C_{1-4}$-alkoxycarbonyl group, —A—$R^5$ or Y and Z form

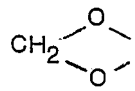 ;

and A represents —O—, —S—, —SO— or —$SO_2$— and $R^5$ represents a halogen-substituted $C_{1-4}$-alkyl group.

2. The pyrazoline derivatives according to Claim 1 which have the formula (I) wherein W represents oxygen atom.

3. The pyrazoline derivatives according to Claim 2 which have the formula (I) wherein $R^1$ represents a $C_{1-4}$-alkyl group, phenyl group, a halogen-substituted phenyl group; and $R^2$ and $R^3$ represent hydrogen atom.

4. The pyrazoline derivatives according to Claim 2 which have the formula (II)

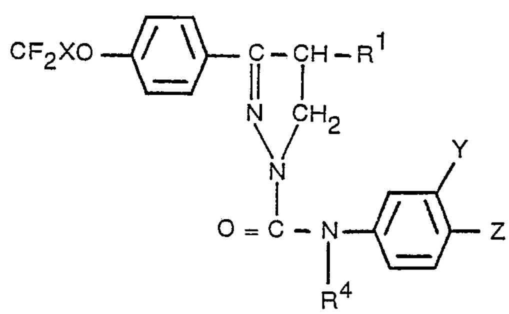

(II)

wherein $R^1$, $R^4$, X, Y and Z have the same meaning as stated in Claim 1.

5. The pyrazoline derivatives according to Claim 4 which have the formula (II) wherein Y represents hydrogen atom or a halogen atom.

6. The pyrazoline derivatives according to Claim 5 which have the formula (II) wherein $R^1$ represents phenyl group or a halogen-substituted phenyl group; $R^4$ represent hydrogen atom.

7. The pyrazoline derivative according to Claim 1, which is 1 - (4 - Chlorophenylcarbamoyl) - 3 - (4 - difluoromethoxyphenyl) - 4 - phenyl-2-pyrazoline.

8. The pyrazoline derivative according to Claim 1, which is 1 - (4 - Trifluoromethylphenylcarbamoyl) - 3 - (4 - difluoromethoxyphenyl) - 4 - phenyl) - 2 - pyrazoline.

9. The pyrazoline derivative according to Claim 1, which is 1 - (4 - Chlorophenylcarbamoyl) - 3 - (4 - difluoromethoxyphenyl) - 4 - (4 - fluorophenyl) - 2 - pyrazoline.

10. The pyrazoline derivative according to Claim 1, which is 1 - (4 - Trifluoromethylphenylcarbamoyl) - 3 - (4 - difluoromethoxyphenyl) - 4 - (4 - fluorophenyl) - 2 - pyrazoline.

11. The pyrazoline derivative according to Claim 1, which is 1 - (4 - Trifluoromethylthiophenyl-carbamoyl) - 3 - (4 - difluoromethoxyphenyl) - 4 - phenyl - 2 - pyrazoline.

12. The pyrazoline derivative according to Claim 1, which is 1 - (4 - Trifluoromethylsulfinylphenyl-carbamoyl) - 3 - (4 - difluoromethoxyphenyl) - 4 - phenyl - 2 - pyrazoline.

13. The pyrazoline derivative according to Claim 1, which is 1 - (4 - Trifluoromethylsulfonylphenyl-carbamoyl) - 3 - (4 - difluoromethoxyphenyl) - 4 - phenyl - 2 - pyrazoline.

14. The pyrazoline derivative according to Claim 1, which is 1 - (4 - Difluoromethoxyphenyl-carbamoyl) - 3 - (4 - difluoromethoxyphenyl) - 4 - phenyl - 2 - pyrazoline.

15. The pyrazoline derivative according to Claim 1, which is 1 - [4-(1',1',2',2'-Tetrafluoro-ethoxy)phenylcarbamoyl)] - 3 - (4 - difluoromethoxyphenyl) - 4 - phenyl - 2 - pyrazoline.

16. The pyrazoline derivative according to Claim 1, which is 1 - (4 - Trifluoromethoxyphenyl-carbamoyl) - 3 - (4 - difluoromethoxyphenyl) - 4 - (4 - chlorophenyl) - 2 - pyrazoline.

17. The pyrazoline derivative according to Claim 1, which is 1 - (4 - Trifluoromethylthiophenyl-carbamoyl) - 3 - (4 - difluoromethoxyphenyl) - 4 - (4 - fluorophenyl) - 2 - pyrazoline.

18. The pyrazoline derivative according to Claim 1, which is 1-(4-Trifluoromethoxyphenylcarbamoyl)-3-(4-difluoromethoxyphenyl)-4-(4-fluorophenyl)-2-pyrazoline.

19. A process for producing a pyrazoline derivative having the formula

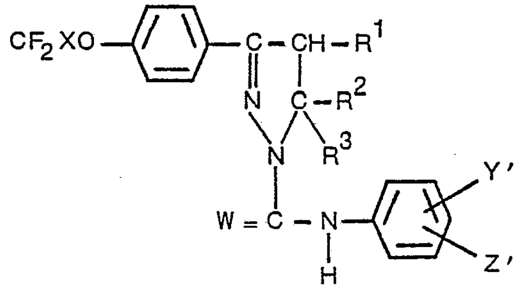

[I′]

wherein $R^1$, $R^2$, $R^3$, X and W are as defined in claim 1; Y′ and Z′ respectively represent hydrogen atom, a halogen atom, a $C_{1-4}$-alkyl group, a $C_{1-4}$-alkoxy group, nitro-group, trifluoromethyl group, a $C_{1-4}$ alkylthio group, an acetyl group, nitrile group, a $C_{1-4}$-alkyl sulfonyl group, a $C_{1-4}$-alkoxycarbonyl group, —A′—$R^5$, or Y′ and Z′ form

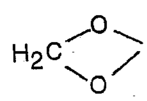

[III]

and A′ represents —O—, —S—, and $R^5$ represents a halogen-substituted $C_{1-4}$-alkyl group which comprises reacting a compound having the formula

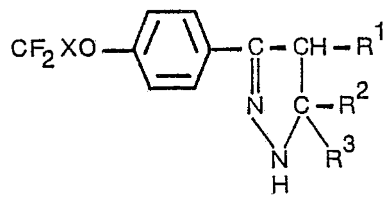

[III]

wherein $R^1$, $R^2$, $R^3$ and X are defined above with a compound having the formula (IV)

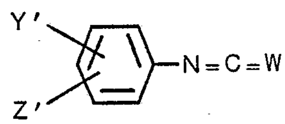

[IV]

wherein W, Y′ and Z′ are defined above.

20. An insecticide which comprises a pyrazoline derivative defined in one of Claims 1 to 18 as an active ingredient.

**Patentansprüche**

1. Pyrazolinderivate der Formel

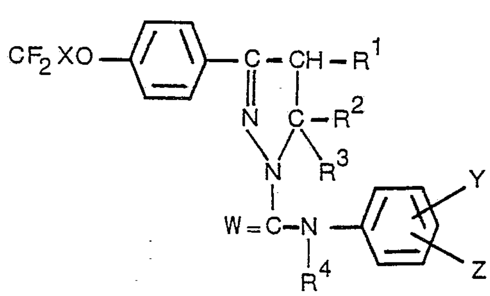

[I]

wobei $R^1$ ein Wasserstoffatom, eine $C_{1-4}$-Alkylgruppe, Phenylgruppe, eine Halogen-substituierte Phenylgruppe, eine Cyanoalkylgruppe oder eine $C_{1-4}$-Alkoxyalkylgruppe bedeutet; $R^2$ und $R^3$ jeweils für Wasserstoffatom, eine $C_{1-4}$-Alkylgruppe, Phenylgruppe oder eine Halogen-substituierte Phenylgruppe

stehen; $R^4$ ein Wasserstoffatom oder eine $C_{1-4}$-Alkylgruppe bedeutet; X für Wasserstoffatom oder ein Halogenatom steht; W ein Sauerstoff- oder Schwefelatom bedeutet; Y und Z bedeuten Jeweils Wasserstoffatom, ein Halogenatom, eine $C_{1-4}$-Alkylgruppe, eine $C_{1-4}$-Alkoxygruppe, Nitrogruppe, Trifluoromethylgruppe, eine $C_{1-4}$-Alkylthiogruppe, eine Acetylgruppe, Nitrilgruppe, eine $C_{1-4}$-Alkylsulfonylgruppe, eine $C_{1-4}$-Alkoxycarbonylgruppe, —A—R oder Y und Z bilden

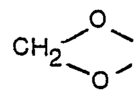 ;

und A bedeutet —O—, —S—, —SO— oder —$SO_2$— und $R^5$ bedeutet eine Halogensubstituierte $C_{1-4}$-Alkylgruppe.

2. Pyrazolinderivate nach Anspruch 1, welche die Formel (I) aufweisen, wobei W für Sauerstoffatom steht.

3. Pyrazolinderivate nach Anspruch 2, welche die Formel (I) aufseisen, wobei $R^1$ eine $C_{1-4}$-Alkylgruppe, Phenylgruppe, eine Halogen-substituierte Phenylgruppe bedeutet und $R^2$ und $R^3$ für Wasserstoffatom stehen.

4. Pyrazolinderivat nach Anspruch 2, welche die Formel (II) aufweisen

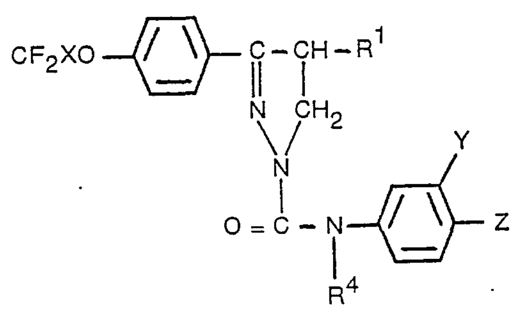

(II)

wobei $R^1$, $R^4$, X, Y und Z die in Anspruch 1 angegebene Bedeutung haben.

5. Pyrazolinderivat nach Anspruch 4, welche die Formel (II) aufweisen, wobei Y für Wasserstoffatom oder ein Halogenatom steht.

6. Pyrazolinderivat nach Anspruch 5, welche die Formel (II) aufweisen, wobei $R^1$ für eine Phenylgruppe oder eine Halogensubstituierte Phenylgruppe steht und $R^4$ für Wasserstoffatom steht.

7. Pyrazolinderivat nach Anspruch 1, nämlich 1 - (4 - Chlorphenylcarbamoyl) - 3 - (4 - difluoromethoxyphenyl) - 4 - phenyl - 2 - pyrazolin.

8. Pyrazolinderivat nach Anspruch 1, nämlich 1 - (4 - Trifluormethylphenylcarbamoyl) - 3 - (4 - difluoromethoxyphenyl) - 4 - phenyl - 2 - pyrazolin.

9. Pyrazolinderivat nach Anspruch 1, nämlich 1 - (4 - Chlorphenylcarbamoyl) - 3 - (4 - difluoromethoxyphenyl) - 4 - (4 - fluorophenyl) - 2 - pyrazolin.

10. Pyrazolinderivat nach Anspruch 1, nämlich 1 - (4 - Trifluormethylphenylcarbamoyl) - 3 - (4 - difluoromethoxyphenyl) - 4 - (4 - fluorphenyl) - 2 - pyrazolin.

11. Pyrazolinderivat nach Anspruch 1, nämlich 1 - (4 - Trifluormethylthiophenylcarbamoyl) - 3 - (4 - difluoromethoxyphenyl) - 4 - phenyl - 2 - pyrazolin.

12. Pyrazolinderivat nach Anspruch 1, nämlich 1 - (4 - Trifluormethylsulfinylphenylcarbamoyl) - 3 - (4 - difluoromethoxyphenyl) - 4 - phenyl - 2 - pyrazolin.

13. Pyrazolinderivat nach Anspruch 1, nämlich 1 - (4 - Trifluormethylsulfonylphenylcarbamoyl) - 3 - (4 - difluoromethoxyphenyl) - 4 - phenyl - 2 - pyrazolin.

14. Pyrazolinderivat nach Anspruch 1, nämlich 1 - (4 - Difluormethoxyphenylcarbamoyl) - 3 - (4 - difluoromethoxyphenyl) - 4 - phenyl - 2 - pyrazolin.

15. Pyrazolinderivat nach Anspruch 1, nämlich 1 - [4 - (1′,1′,2′,2′-Tetrafluorethoxy)phenylcarbamoyl] - 3 - (4 - difluoromethoxyphenyl) - 4 - phenyl - 2 - pyrazolin.

16. Pyrazolinderivat nach Anspruch 1, nämlich 1 - (4 - Trifluormethoxyphenylcarbamoyl) - 3 - (4 - difluoromethoxyphenyl) - 4 - (4 - chlorphenyl) - 2 - pyrazolin.

17. Pyrazolinderivat nach Anspruch 1, nämlich 1 - (4 - Trifluormethylthiophenylcarbamoyl) - 3 - (4 - difluoromethoxyphenyl) - 4 - 4 - fluorphenyl) - 2 - pyrazolin.

18. Pyrazolinderivat nach Anspruch 1, nämlich 1 - (4 - Trifluormethoxyphenylcarbamoyl) - 3 - (4 - difluoromethoxyphenyl) - 4 - (4 - fluorphenyl) - 2 - pyrazolin.

19. Verfahren zur Herstellung eines Pyrazolinderivates der Formel

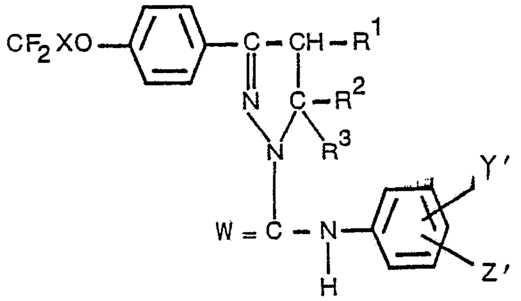

[I']

wobei $R^1$, $R^2$, $R^3$, X und W die in Anspruch 1 angegebene Bedeutung haben; Y' und Z' jeweils für Wasserstoffatom, ein Halogenatom, eine $C_{1-4}$-Alkylgruppe, eine $C_{1-4}$-Alkoxygruppe, Nitrogruppe, Trifluormethylgruppe, eine $C_{1-4}$-Alkylthiogruppe, eine Acetylgruppe, Nitrilgruppe, eine $C_{1-4}$-Alkylsulfonylgruppe, eine $C_{1-4}$-Alkoxycarbonylgruppe, —A'—$R^5$ stehen oder Y' und Z'

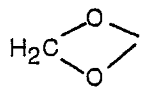

bilden und A' für —O—, —S— steht und $R^5$ eine Halogen-substituierte $C_{1-4}$-Alkylgruppe bedeutet, umfassend die Umsetzung einer Verbindung der Formel

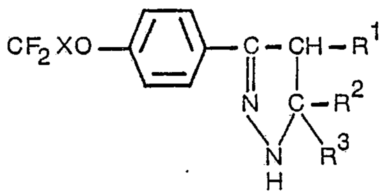

wobei $R^1$, $R^2$, $R^3$ und x die oben angegebene Bedeutung haben, mit einer Verbindung der Formel (IV)

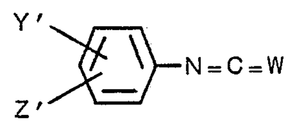

[IV]

wobei W, Y' und Z' die oben angegebene Bedeutung haben.

20. Ein Insektizid, welches ein Pyrazolinderivat nach einem der Ansprüche 1 bis 18 als Wirkstoff enthält.

**Revendications**

1. Dérivés de la pyrazoline répondant à la formule

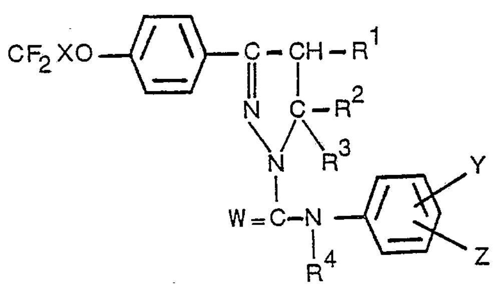

[I]

dans laquelle $R^1$ représente l'atome d'hydrogène, un groupe alkyle en $C_{1-4}$, le groupe phényle, un groupe phényle substitué par de l'halogène, un groupe cyano alkyle ou un groupe alcoxy (en $C_{1-4}$) alkyle; $R^2$ et $R^3$ représentent respectivement l'atome d'hydrogène, un groupe alkyle en $C_{1-4}$, le groupe phényle ou un

groupe phényle substitué par de l'halogène; $R^4$ représente l'atome d'hydrogène ou un groupe alkyle en $C_{1-4}$; X représente l'atome d'hydrogène ou un atome d'halogène; W représente l'atome d'oxyugène ou de soufre; Y et Z représentent respectivement l'atome d'hydrogène, un atome d'halogéne, un groupe alkyle en $C_{1-4}$, un groupe alcoxy en $C_{1-4}$, le groupe nitro, le groupe trifluorométhyle, un groupe alkylthio en $C_{1-4}$, un groupe acétyle, le groupe nitrile, un groupe alkyl sulfinyl en $C_{1-4}$, un groupe alcoxy(en $C_{1-4}$) carbonyle, —A—$R^5$ ou Y et Z forment

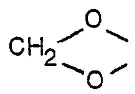 ;

et A représente —O—, —S—, —SO— ou —$SO_2$— et $R^5$ représente un groupe alkyle en $C_{1-4}$ substitué par de l'halogène.

2. Les dérivés de la pyrazoline selon la revendication 1 qui possèdent la formule (I) dans laquelle W représente l'atome d'oxygène.

3. Les dérivés de la pyrazoline selon la revendication 2 qui possèdent la formule (I) dans laquelle $R^1$ représente un groupe alkyle en $C_{1-4}$, le groupe phényle, un groupe phényle substitué par de l'halogène; et $R^2$ et $R^3$ représentent l'atome d'hydrogène.

4. Les dérivés de la pyrazoline selon la revendication 2 qui possèdent la formule (II)

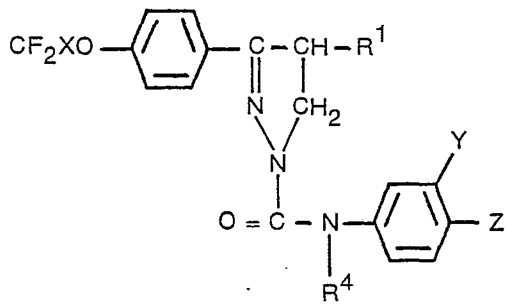

(II)

dans laquelle $R^1$, $R^4$, X, Y et Z ont la même signification que donnée dans la revendication 1.

5. Les dérivés de la pyrazoline selon la revendication 4 qui possèdent la formule (II) dans laquelle Y représente l'atome d'hydrogène ou un atome d'halogène.

6. Les dérivés de la pyrazoline selon la revendication 5 qui possèdent la formule (II) dans laquelle $R^1$ représente le groupe phényle ou un groupe phényle substitué par de l'halogène; $R^4$ représente l'atome d'hydrogène.

7. Le dérivé de la pyrazoline selon la revendication 1, qui est la 1 - (4 - chlorophénylcarbamoyl) - 3 - (4 - difluorométhoxyphényl- - 4 - phényl - 2 - pyrazoline.

8. Le dérivé de la pyrazoline selon la revendication 1, qui est la 1 - (4 - Trifluorométhylphénylcarbamoyl) - 3 - (4 - difluorométhoxyphényl- - 4 - phényl - 2 - pyrazoline.

9. Le dérivé de la pyrazoline selon la revendication 1, qui est la 1 - (4 - Chlorophénylcarbamoyl) - 3 - (4 - difluorométhoxyphényl - 4 - (4-fluorophényl) - 2 - pyrazoline.

10. Le dérivé de la pyrazoline selon la revendication 1, qui est la 1 - (4 - Trifluorométhylphénylcarbamoyl) - 3 - (4 - difluorométhoxyphényl - 4 - (4 - fluorophényl - 2 - pyrazoline.

11. Le dérivé de la pyrazoline selon la revendication 1, qui est la 1 - (4 - Trifluorométhylthiophénylcarbamoyl) - 3 - (4 - difluorométhoxyphényl) - 4 - phényl - 2 - pyrazoline.

12. Le dérivé de la pyrazoline selon la revendication 1, qui est la 1 - (4 - Trifluorométhylsulfinylphénylcarbamoyl) - 3 - (4 - difluorométhoxyphényl - 4 - phényl - 2 - pyrazoline.

13. Le dérivé de la pyrazoline selon la revendication 1, qui est la 1 - (4 - Trifluorométhylsulfonylphénylcarbamoyl) - 3 - (4 - difluorométhoxyphényl- - 4 - phényl - 2 - pyrazoline.

14. Le dérivé de la pyrazoline selon la revendication 1, qui est la 1 - (4 - Difluorométhoxyphénylcarbamoyl) - 3 - (4 - difluorométhoxyphényl) - 4 - phényl - 2 - pyrazoline.

15. Le dérivé de la pyrazoline selon la revendication 1, qui est la 1 - (4 - (1',1',2'2'-Tétrafluorométhoxy)phénylcarbamoyl) - 3 - (4 - difluorométhoxyphényl) - 4 - phényl - 2 - pyrazoline.

16. Le dérivé de la pyrazoline selon la revendication 1. qui est la 1 - (4 - Trifluorométhoxyphénylcarbamoyl) - 3 - (4 - difluorométhoxyphényl) - 4 - (4 - chlorophényl - 2 - pyrazoline.

17. Le dérivé de la pyrazoline selon la revendication 1, qui est la 1 - (4 - Trifluorométhylthiophénylcarbamoyl) - 3 - (4 - difluorométhoxyphényl) - 4 - (4 - fluorophényl - 2 - pyrazoline.

18. Le dérivé de la pyrazoline selon la revendication 1, qui est la 1 - (4 - Trifluorométhoxyphénylcarbamoyl) - 3 - (4 - difluorométhoxyphényl) - 4 - (4 - fluorophényl - 2 - pyrazoline.

19. Un procédé pour la production d'un dérivé de la pyrazoline répondant à la formule

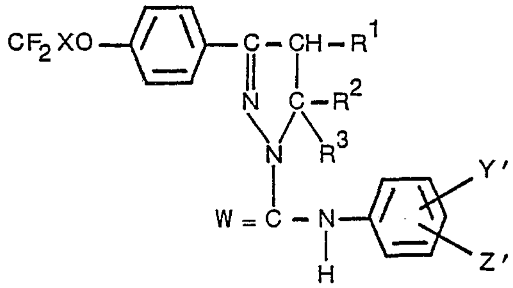

[I']

dans laquelle $R^1$, $R^2$, $R^3$ X et W sont tels que définis dans la revendication 1; Y' et Z' représentent respectivement l'atome d'hydrogène, un atome d'halogéne, un groupe alkyle en $C_{1-4}$, un groupe alcoxy en $C_{1-4}$, le groupe nitro, le groupe trifluorométhyle, un groupe alkylthio en $C_{1-4}$, un groupe acétyle, le groupe nitrile, un groupe alkyl sulfonyle en $C_{1-4}$, un groupe alcoxy (en $C_{1-4}$) carbonyle, —A'—$R^5$, ou Y' et Z' forment

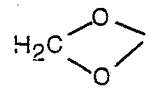

et A' représent —O—, —S—, et $R^5$ représente un groupe alkyle en $C_{1-4}$ substitué par de l'halogéne, qui consiste à faire réagir un composé répondant à la formule

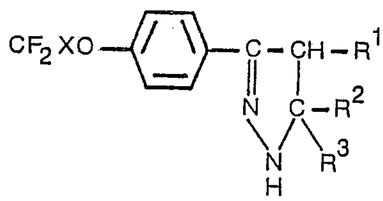

[III]

dans laquelle $R^1$, $R^2$, $R^3$ et X sont tels que définis ci-dessus avec un composé répondant à la formule (IV)

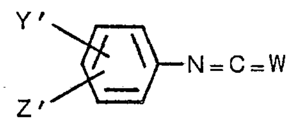

[IV]

dans laquelle W, Y' et Z' sont définis ci-dessus.

20. Un insecticide qui comprend un dérivé de la pyrazoline défini dans l'une des revendications 1 à 13 à titre d'ingrédient actif.